(19) Europäisches Patentamt
European Patent Office
Office européen des brevets

(11) Veröffentlichungsnummer: **0 428 928 A2**

(12) **EUROPÄISCHE PATENTANMELDUNG**

(21) Anmeldenummer: **90121078.1**

(22) Anmeldetag: **03.11.90**

(51) Int. Cl.5: **C07C 255/64**, A01N 37/34, A01N 43/08, A01N 43/36, C07D 207/22, C07D 209/48, C07D 263/22, C07D 295/13, C07D 307/52

(30) Priorität: **17.11.89 DE 3938287**

(43) Veröffentlichungstag der Anmeldung: **29.05.91 Patentblatt 91/22**

(84) Benannte Vertragsstaaten: **BE CH DE ES FR GB GR IT LI NL**

(71) Anmelder: **BAYER AG**
**W-5090 Leverkusen 1 Bayerwerk(DE)**

(72) Erfinder: **Lunkenheimer, Winfried, Dr.**
**Funckstrasse 49**
**W-5600 Wuppertal 1(DE)**
Erfinder: **Brandes, Wilhelm, Dr.**
**Eichendorffstrasse 3**
**W-5653 Leichlingen 1(DE)**

# (54) Substituierte Aminale.

(57) Beschrieben werden neue substituierte Aminale der Formel (I)

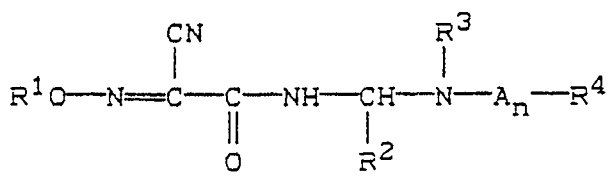

(I)

in welcher $R^1$, $R^2$, $R^3$, $R^4$, A und n die in der Beschreibung angegebene Bedeutung haben sowie mehrere Verfahren zu ihrer Herstellung.

Die neuen Aminale der Formel (I) werden zur Bekämpfung von Schädlingen verwendet.

EP 0 428 928 A2

## SUBSTITUIERTE AMINALE

Die vorliegende Erfindung betrifft neue substituierte Aminale, mehrere Verfahren zu ihrer Herstellung und ihre Verwendung in Schädlingsbekämpfungsmitteln, insbesondere als Fungizide.

Es ist bereits bekannt, daß bestimmte substituierte 2-Cyano-2-oximino-acetamide gute fungizide Wirksamkeit besitzen (vergleiche z. B. EP 0 201 999 und DE-OS 23 12 956 und DE-OS 36 02 243). Die Wirkung dieser Verbindungen ist jedoch, insbesondere bei niedrigen Aufwandmengen und -konzentrationen, nicht immer voll befriedigend.

Es wurden neue substituierte Aminale der allgemeinen Formel (I)

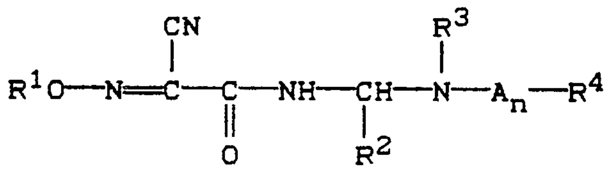

(I)

in welcher

A für -CO-, -CS- oder $-SO_2-$ steht,

n für 0 oder 1 steht,

$R^1$ für gegebenenfalls substituiertes Alkyl steht, wobei als Substituenten genannt seien: Halogen, Cyano, $-COOR^I$, $-CONR^{II}R^{III}$, $-OR^{IV}$, Acyl, gegebenenfalls substituiertes Aryl, jeweils gegebenenfalls substituiertes Cycloalkyl oder Heterocyclyl;

$R^1$ ferner für jeweils gegebenenfalls substituiertes Alkenyl, Alkinyl, Cycloalkyl oder Cycloalkenyl steht,

$R^2$ für Wasserstoff oder für gegebenenfalls substituiertes Alkyl steht, wobei als Substituenten genannt seien: Cyano, Alkoxy, Alkylthio, $-COOR^I$, Acylamino sowie jeweils gegebenenfalls substituiertes Aryl, Cycloalkyl oder Heterocyclyl;

$R^2$ ferner für jeweils gegebenenfalls substituiertes Alkenyl, Alkinyl, Cycloalkyl oder Cycloalkenyl sowie für jeweils gegebenenfalls substituiertes Aryl oder Heterocyclyl steht,

$R^3$ für gegebenenfalls substituiertes Alkyl steht, wobei als Substituenten genannt seien: Halogen, Cyano, $-COOR^I$, $-CONR^{II}R^{III}$, $-OR^{IV}$, die Oxo- oder Thioxogruppe sowie jeweils gegebenenfalls substituiertes Aryl, Cycloalkyl oder Heterocyclyl; ferner für jeweils gegebenenfalls substituiertes Alkenyl, Alkinyl, Cycloalkyl oder Aryl oder für gegebenenfalls substituiertes und gegebenenfalls anelliertes Heterocyclyl sowie für $-OR^V$ steht; weiterhin

$R^3$ für Wasserstoff steht,

für den Fall daß entweder n = 1 ist und A für $-SO_2-$steht oder n = 1 ist und $R^4$ für Cycloalkoximino oder Arylthio steht,

$R^4$ für Wasserstoff oder für gegebenenfalls substituiertes Alkyl steht, wobei als Substituenten genannt seien: Halogen, Alkoxy, Alkylthio, Alkylsulfinyl, Alkylsulfonyl, Acyloxy, Acylamino, Cyano, $-COOR^I$, jeweils gegebenenfalls substituiertes Aryl, Cycloalkyl oder Heterocyclyl; ferner für jeweils gegebenenfalls substituiertes Alkenyl oder Alkinyl steht; $R^4$ weiterhin für jeweils gegebenenfalls überbrücktes und/oder anelliertes, jeweils gegebenenfalls substituiertes Cycloalkyl oder Cycloalkenyl steht sowie für jeweils gegebenenfalls anelliertes, jeweils gegebenenfalls substiuiertes Aryl oder Heterocyclyl, für jeweils gegebenenfalls substituiertes Aryloxy oder Arylthio sowie für Alkoxycarbonyl, für 1-Cyanoalkoximino, $-OR^5$, $-SR^5$ und $-NR^6R^7$ steht, oder

$R^3$ und $R^4$ gemeinsam mit dem Stickstoffatom und gegebenenfalls dem Rest A einen gegebenenfalls substituierten 4- bis 9-gliedrigen, gesättigten oder ungesättigten heterocyclischen Ring bilden, welcher gegebenenfalls weitere Heteroatome aus der Reihe Sauerstoff, Schwefel, Stickstoff enthält und welcher gegebenenfalls durch 1 oder 2 gesättigte oder ungesättigte Carbocyclen anelliert ist,

$R^5$ für Wasserstoff, für gegebenenfalls substituiertes Alkyl steht, wobei als Substituenten genannt seien: Halogen, $-OR^{IV}$, $-SR^{IV}$, $-COOR^I$, $-CONR^{II}R^{III}$, -CN, $-NR^{II}R^{III}$, Acyl, jeweils gegebenenfalls substituiertes Aryl oder Aryloxy, jeweils gegebenenfalls substituiertes Cycloalkyl, Cycloalkenyl oder Heterocyclyl; ferner für jeweils gegebenenfalls substituiertes Alkenyl oder Alkinyl sowie für jeweils gegebenen substituiertes Cycloalkyl oder Cycloalkenyl steht,

$R^6$ für Wasserstoff oder für gegebenenfalls substituiertes Alkyl steht, wobei als Substituenten genannt seien: Halogen, Cyano, $-COOR^I$, $-CON^{II}R^{III}$, $-NR^{II}R^{III}$, $-OR^{IV}$, $-S(O)_nR^{IV}$, Acyl, jeweils gegebenenfalls substituiertes Aryl, Heterocyclyl, Cycloalkyl oder Cycloalkenyl; ferner für jeweils gegebenenfalls substituiertes Alkenyl, Alkinyl, Cycloalkyl, Cycloalkenyl, Aryl oder Heterocyclyl steht und

$R^7$ für die Bedeutung von $R^6$ oder für -$OR^{IV}$ steht, oder
$R^6$ und $R^7$ gemeinsam mit dem Stickstoffatom, an das sie gebunden sind, für einen gegebenenfalls substituierten Heterocyclus stehen, der weitere Heteroatome enthalten kann,
$R^I$ für Wasserstoff oder gegebenenfalls substituiertes Alkyl steht mit folgenden Substituenten: Halogen, jeweils gegebenenfalls substituiertes Aryl, Cycloalkyl und Cycloalkenyl,
$R^{II}$ und $R^{III}$ gleich oder verschieden sind und für Wasserstoff oder gegebenenfalls substituiertes Alkyl stehen mit folgenden Substituenten: Halogen, -$COOR^{IV}$, -$CONR^IR^{IV}$, jeweils gegebenenfalls substituiertes Aryl, Aralkyl, Cycloalkyl und Cycloalkenyl; $R^{II}$ und $R^{III}$ ferner für Alkenyl, Alkinyl oder jeweils gegebenenfalls substituiertes Cycloalkyl, Cycloalkenyl, Aryl oder Heterocyclyl stehen oder gemeinsam mit dem Stickstoffatom, an das sie gebunden sind, für einen gegebenenfalls substituierten Heterocyclus stehen, der weitere Heteroatome enthalten kann,
$R^{IV}$ für Wasserstoff, Alkyl, Aralkyl oder Acyl steht und
$R^V$ für Wasserstoff, Alkyl oder Aralkyl steht,
gefunden.

Die Verbindungen der Formel (I) können in verschiedenen geometrischen Isomeren vorkommen, je nach Anordnung der Substituenten an der C=N-Gruppierung (E- bzw. Z-Isomere). Sowohl die reinen Z- und E-Isomeren, als auch deren Gemische werden erfindungsgemäß beansprucht.

Gegebenenfalls besitzen die Verbindungen ein oder mehrere asymmetrische Kohlenstoffatome; sie können somit auch als Enantiomere oder Diastereomere vorliegen, die in unterschiedlichen Mengenverhältnissen anfallen können. Vorwiegend fallen sie als Racemate an. Sowohl die reinen Enantiomeren und Diastereomeren, als auch die Gemische werden erfindungsgemäß beansprucht.

Im folgenden wird der Einfachheit halber stets von Verbindungen der Formel (I) gesprochen, obwohl sowohl die reinen Verbindungen als auch die Gemische mit unterschiedlichen Anteilen an isomeren, enantiomeren und diastereomeren Verbindungen gemeint sind.

Weiterhin wurde gefunden, das man die neuen substituierten Aminale der allgemeinen Formel (I),

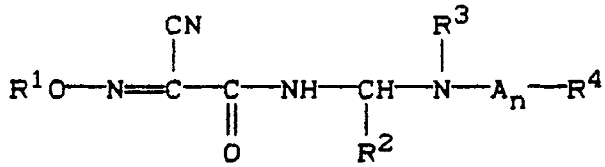

(I)

in welcher
A, n, $R^1$, $R^2$, $R^3$ und $R^4$ die oben angegebene Bedeutung haben,
erhält, wenn man

a) 2-Cyano-2-oximinocarbonyl-Verbindungen der Formel (II)

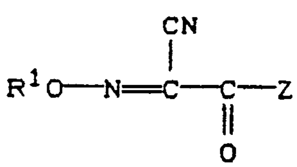

(II)

in welcher
$R^1$ die oben angegebene Bedeutung hat und
Z für Chlor, Methoxy oder Ethoxy steht
mit Amino-Verbindungen der Formel (III)

HY x

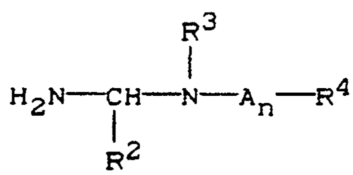

(III)

in welcher
$R^2$, $R^3$, $R^4$, A und n die oben angegebene Bedeutung haben und
HY für das Äquivalent einer anorganischen oder organischen Säure steht,
gegebenenfalls in Gegenwart einer Base und in Gegenwart eines Verdünnungsmittels und gegebenenfalls in Gegenwart eines Katalysators umsetzt;
oder
b) 2-Cyano-2-oximinoacetamide der Formel (IV)

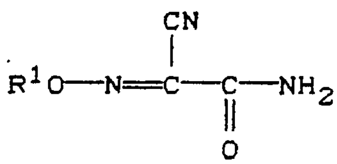

(IV)

in welcher
$R^1$ die oben angegebene Bedeutung hat,
mit Verbindungen der Formel (V),

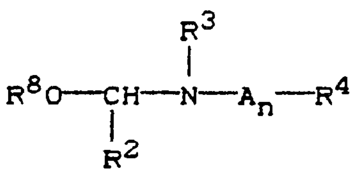

(V)

in welcher
$R^2$, $R^3$, $R^4$, A und n die oben angegebene Bedeutung haben und
$R^8$ für Wasserstoff, Methyl oder Acetyl steht,
gegebenenfalls in Gegenwart einer Säure und gegebenenfalls in Gegenwart eines Verdünnungsmittels umsetzt;
oder
c) 2-Cyano-2-oximinoacetamide der Formel (IV)

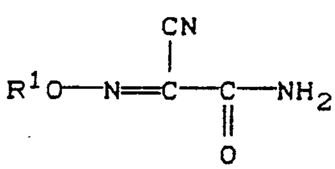

(IV)

in welcher
$R^1$ die oben angegebene Bedeutung hat,
mit Halogen-Verbindungen der Formel (VI)

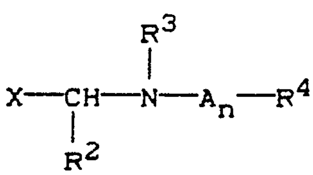

(VI)

in welcher
$R^2$, $R^3$, $R^4$, A und n die oben angegebene Bedeutung haben und
X für Chlor oder Brom steht,
gegebenenfalls in Gegenwart einer Base und gegebenenfalls in Gegenwart eines Verdünnungsmittels umsetzt.
Man erhält die erfindungsgemäßen Verbindungen der Formel (Ia)

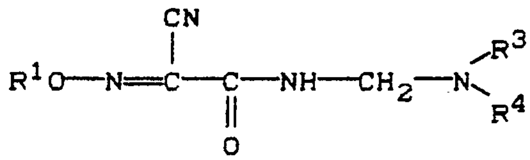

(Ia)

in welcher
$R^1$, $R^3$ und $R^4$ die oben angegebene Bedeutung haben,
wenn man
d) 2-Cyano-2-oximinoacetamide der Formel (IV)

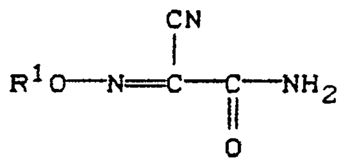

(IV)

in welcher
$R^1$ die oben angegebene Bedeutung hat,
mit Aminen der Formel (VII)

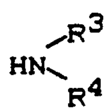

(VII)

in welcher
$R^3$ und $R^4$ die oben angegebene Bedeutung haben
und mit Formaldehyd
gegebenenfalls in Gegenwart eines Verdünnungsmittels umsetzt.
Man erhält die erfindungsgemäßen Verbindungen der Formel (Ib)

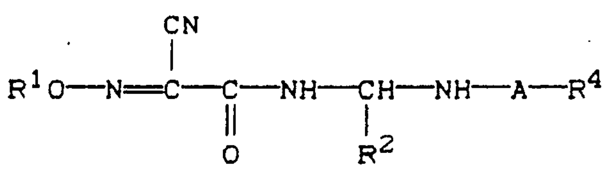

(Ib)

in welcher
$R^1$, $R^2$, $R^4$ und A die oben angegebene Bedeutung haben, wenn man
e) N-(2-Cyano-2-oximino-acetyl)-aminale der Formel (VIII)

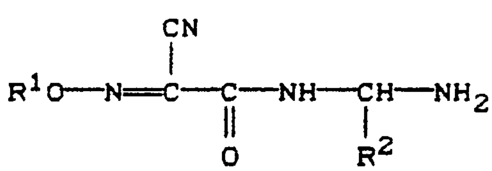 x HY (VIII)

in welcher
$R^1$ und $R^2$ die oben angegebene Bedeutung haben,
und
HY für das Äquivalent einer anorganischen oder organischen Säure steht,
mit einem Acylierungsreagenz der Formel (IX)
Q-A-$R^4$ (IX)

in welcher

$R^4$ und A die oben angegebene Bedeutung haben und

Q für eine übliche Abgangsgruppe steht, wie Halogen, Alkoxy, Alkylthio, $-O-COR^4$, $-O-COOR^5$, $-OR^5$, $-SR^5$, Carboxymethoxy oder Carboxymethylthio steht,

gegebenenfalls in Gegenwart einer Base und in Gegenwart eines Verdünnungsmittels und gegebenenfalls in Gegenwart eines Katalysators umsetzt.

Schließlich wurde gefunden, daß die neuen substituierten Aminale unter anderem starke fungizide Eigenschaften aufweisen. Dabei zeigen überraschenderweise die erfindungsgemäßen Verbindungen eine höhere Wirkung als die aus dem Stand der Technik bekannten substituierten 2-Cyano-2-oximinoacetamide, welche konstitutionell bzw. wirkungsmäßig naheliegende Verbindungen sind. Die erfindungsgemäßen Stoffe stellen somit eine Bereicherung der Technik dar.

Im folgenden können alle aliphatischen Reste, wie Alkyl, Alkoxy, Alkenyl usw., allein oder in Zusammensetzungen, wie Alkoxyalkyl, geradkettig oder verzweigt sein, weiterhin können die aliphatischen Reste im allgemeinen vorzugsweise ein- bis fünffach, besonders bevorzugt einbis dreifach oder ganz besonders bevorzugt ein- oder zweifach, gleich oder verschieden substituiert sein; ebenso sind alle Ringsysteme gegebenenfalls ein- bis fünffach, besonders bevorzugt ein- bis dreifach oder ganz besonders bevorzugt ein- oder zweifach, gleich oder verschieden substituiert, wenn es nicht anders angegeben oder nicht ausdrücklich beschrieben ist.

Die erfindungsgemäßen substituierten Aminale sind durch die Formel (I) allgemein definiert. In dieser Formel (I) stehen vorzugsweise

A für -CO-, -CS- oder $-SO_2-$,

n für 0 oder 1,

$R^1$ für gegebenenfalls ein- bis fünffach, gleich oder verschieden substituiertes, geradkettiges oder verzweigtes Alkyl mit 1 bis 6 Kohlenstoffatomen, wobei folgende Substituenten infrage kommen: Fluor, Chlor, Brom, Iod, Cyano, $-COOR^I$, $-CONR^{II}R^{III}$, $-OR^{IV}$, Acyl mit 2 bis 9 Kohlenstoffatomen, gegebenenfalls ein- bis fünffach, gleich oder verschieden substituiertes Phenyl, wobei als Phenylsubstituenten genannt seien: Halogen, Alkyl und Alkoxy mit jeweils 1 bis 4 Kohlenstoffatomen, Halogenalkyl und Halogenalkoxy mit jeweils 1 oder 2 Kohlenstoffatomen und 1 bis 5 gleichen oder verschiedenen Halogenatomen; weitere Alkylsubstituenten sind gegebenenfalls ein- bis fünffach, gleich oder verschieden durch Alkyl mit 1 bis 4 Kohlenstoffatomen substituiertes Cycloalkyl mit 3 bis 6 Kohlenstoffatomen, gegebenenfalls ein- bis fünffach, gleich oder verschieden durch Halogen und Alkyl mit 1 bis 4 Kohlenstoffatomen substituiertes und gegebenenfalls durch 1 oder 2 6-gliedrige gesättigte oder ungesättigte Carbocyclen anelliertes 3- bis 6-gliedriges Heterocyclyl mit 1 bis 3 gleichen oder verschiedenen Heteroatomen, wie Stickstoff-, Sauerstoff- und Schwefelatomen; $R^1$ steht ferner vorzugsweise für jeweils gegebenenfalls ein- bis dreifach, gleich oder verschieden durch Alkyl mit 1 bis 4 Kohlenstoffatomen substituiertes, geradkettiges oder verzweigtes Alkenyl oder Alkinyl mit 2 bis 6 Kohlenstoffatomen; $R^1$ steht außerdem vorzugsweise für jeweils gegebenenfalls ein- bis fünffach, gleich oder verschieden durch Alkyl mit 1 bis 4 Kohlenstoffatomen substituiertes Cycloalkyl mit 3 bis 6 Kohlenstoffatomen oder Cycloalkenyl mit 5 bis 7 Kohlenstoffatomen;

$R^2$ für Wasserstoff oder für gegebenenfalls ein- bis fünffach, gleich oder verschieden substituiertes, geradkettiges oder verzweigtes Alkyl mit 1 bis 6 Kohlenstoffatomen, wobei folgende Substituenten infrage kommen: Cyano, Alkoxy und Alkylthio mit jeweils 1 bis 4 Kohlenstoffatomen, $-COOR^I$, Acylamino mit 2 bis 9 Kohlenstoffatomen, gegebenenfalls ein- bis fünffach, gleich oder verschieden substituiertes Phenyl, wobei als Substituenten die bei $R^1$ bereits vorzugsweise genannten Phenylsubstituenten infrage kommen; weitere Alkylsubstituenten sind gegebenenfalls ein- bis fünffach, gleich oder verschieden durch Alkyl mit 1 bis 4 Kohlenstoffatomen substituiertes Cycloalkyl mit 3 bis 6 Kohlenstoffatomen, gegebenenfalls ein- bis fünffach, gleich oder verschieden durch Halogen und Alkyl mit 1 bis 4 Kohlenstoffatomen substituiertes und gegebenenfalls durch 1 oder 2 6-gliedrige, gesättigte oder ungesättigte Carbocyclen anelliertes 3- bis 6-gliedriges Heterocyclyl mit 1 bis 3 gleichen oder verschiedenen Heteroatomen, wie Stickstoff-, Sauerstoff- und Schwefelatomen; $R^2$ steht ferner vorzugsweise für jeweils gegebenenfalls ein- bis dreifach, gleich oder verschieden substituiertes, geradkettiges oder verzweigtes Alkenyl oder Alkinyl mit 2 bis 6 Kohlenstoffatomen, wobei als Substituenten genannt seien: gegebenenfalls ein- bis fünffach, gleich oder verschieden durch Halogen und Alkyl mit 1 bis 4 Kohlenstoffatomen substituiertes Phenyl; $R^2$ steht außerdem vorzugsweise für jeweils gegebe nenfalls ein- bis fünffach, gleich oder verschieden durch Alkyl mit 1 bis 4 Kohlenstoffatomen substituiertes Cycloalkyl mit 3 bis 6 Kohlenstoffatomen oder Cycloalkenyl mit 5 bis 7 Kohlenstoffatomen; $R^2$ steht weiterhin vorzugsweise für gegebenenfalls ein- bis fünffach, gleich oder verschieden substituiertes Phenyl, wobei als Substituenten die bei $R^1$ bereits vorzugsweise genannten Phenylsubstituenten infrage kommen; $R^2$ steht ferner vorzugsweise für gegebenenfalls ein-bis fünffach, gleich oder verschieden durch Halogen und Alkyl mit 1 bis 4 Kohlenstoffatomen substituiertes und

gegebenenfalls durch 1 oder 2 6-gliedrige, gesättigte oder ungesättigte Carbocyclen anelliertes 3- bis 6-gliedriges Heterocyclyl mit 1 bis 3 gleichen oder verschiedenen Heteroatomen, wie Stickstoff-, Sauerstoff- und Schwefelatome;

$R^3$ für gegebenenfalls ein- bis fünffach, gleich oder verschieden substituiertes, geradkettiges oder verzweigtes Alkyl mit 1 bis 6 Kohlenstoffatomen, wobei folgende Substituenten infrage kommen: Fluor, Chlor, Brom, Iod, Cyano, $-COOR^{I}$, $-CONR^{II}R^{III}$, $-OR^{IV}$, die Oxo- oder Thioxogruppe, gegebenenfalls ein- bis fünffach, gleich oder verschieden substituiertes Phenyl, wobei als Substituenten die bei $R^1$ bereits vorzugsweise genannten Phenylsubstituenten infrage kommen; weitere Alkylsubstituenten sind gegebenenfalls ein- bis fünffach, gleich oder verschieden durch Alkyl mit 1 bis 4 Kohlenstoffatomen substituiertes Cycloalkyl mit 3 bis 6 Kohlenstoffatomen sowie gegebenenfalls ein- bis fünffach, gleich oder verschieden durch Halogen, durch Alkyl, Alkoxy oder Alkylthio mit jeweils 1 bis 4 Kohlenstoffatomen, durch Hydroxy, die Oxo- oder Thioxogruppe, Carboxy, Alkoxycarbonyl mit 1 bis 4 Kohlenstoffatomen im Alkoxyteil oder durch Carbamoyl substituiertes 3- bis 6-gliedriges Heterocyclyl mit 1 bis 3 gleichen oder verschiedenen Heteroatomen, wie Stickstoff-, Sauerstoff- und Schwefelatomen; $R^3$ steht ferner vorzugsweise für jeweils gegebenenfalls ein- bis dreifach, gleich oder verschieden durch Halogen oder Alkyl mit 1 bis 4 Kohlenstoffatomen substituiertes, geradkettiges oder verzweigtes Alkenyl oder Alkinyl mit jeweils 2 bis 6 Kohlenstoffatomen; $R^3$ steht außerdem vorzugsweise für jeweils gegebenenfalls ein- bis fünffach, gleich oder verschieden durch Alkyl mit 1 bis 4 Kohlenstoffatomen substituiertes Cycloalkyl mit 3 bis 6 Kohlenstoffatomen; für gegebenenfalls ein-bis fünffach, gleich oder verschieden substituiertes Phenyl, wobei als Substituenten die bei $R^1$ bereits vorzugsweise genannten Phenylsubstituenten infrage kommen; $R^3$ steht ferner vorzugsweise für gegebenenfalls ein- bis fünffach, gleich oder verschieden durch Halogen, durch Alkyl, Alkoxy oder Alkylthio mit jeweils 1 bis 4 Kohlenstoffatomen, durch Hydroxy, Mercapto, die Oxo- oder Thioxogruppe, durch Carboxy oder Alkoxycarbonyl mit 1 bis 4 Kohlenstoffatomen im Alkoxyteil sowie durch Carbamoyl substituiertes 3-bis 6-gliedriges Heterocyclyl mit 1 bis 3 gleichen oder verschiedenen Heteroatomen, wie Stickstoff-, Sauerstoff- und Schwefelatome, an welches gegebenenfalls 1 oder 2 6-gliedrige gesättigte oder ungesättigte Carbocyclen anelliert sind und weiterhin für $-OR^{V}$;

außerdem steht

$R^3$ für Wasserstoff,

für den Fall das entweder n = 1 ist und A für $-SO_2$-steht oder n = 1 ist und $R^4$ für Cyanalkoximino oder Arylthio steht,

$R^4$ für Wasserstoff oder für gegebenenfalls ein- bis fünffach, gleich oder verschieden substituiertes, geradkettiges oder verzweigtes Alkyl mit 1 bis 6 Kohlenstoffatomen, wobei folgende Substituenten infrage kommen: Fluor, Chlor, Brom, Iod, Cyano, Alkoxy, Alkylthio, Alkylsulfinyl und Alkylsulfonyl mit jeweils 1 bis 4 Kohlenstoffatomen, Acyloxy und Acylamino mit jeweils 2 bis 9 Kohlenstoffatomen, $-COOR^{I}$, gegebenenfalls ein- bis fünffach, gleich oder verschieden substituiertes Phenyl, wobei als Substituenten die bei $R^1$ bereits vorzugsweise genannten Phenylsubstituenten infrage kommen; weitere Alkylsubstituenten sind gegebenenfalls ein- bis fünffach, gleich oder verschieden durch Alkyl mit 1 bis 4 Kohlenstoffatomen substituiertes Cycloalkyl mit 3 bis 6 Kohlenstoffatomen sowie gegebenenfalls ein- bis fünffach, gleich oder verschieden durch Halogen und Alkyl mit 1 bis 4 Kohlenstoffatomen substituiertes und gegebenenfalls durch 1 oder 2 6-gliedrige gesättigte oder ungesättigte Carbocyclen, anelliertes 3- bis 6-gliedriges Heterocyclyl mit 1 bis 3 gleichen oder verschiedenen Heteroatomen, wie Stickstoff-, Sauerstoff- und Schwefelatomen; $R^4$ steht ferner vorzugsweise für jeweils gegebenenfalls ein- bis dreifach, gleich oder verschieden substituiertes, geradkettiges oder verzweigtes Alkenyl oder Alkinyl mit jeweils 2 bis 6 Kohlenstoffatomen, wobei als Substituenten genannt seien: Alkyl mit 1 bis 4 Kohlenstoffatomen oder gegebenenfalls ein- bis fünffach, gleich oder verschieden durch Halogen und Alkyl mit 1 bis 4 Kohlenstoffatomen substituiertes Phenyl; $R^4$ steht weiterhin vorzugsweise für jeweils gegebenenfalls mit Methylen oder Ethylen überbrücktes und/oder mit 1 oder 2 Benzol-, Cyclopentan- oder Cyclohexanringen anelliertes, jeweils gegebenenfalls ein- bis fünffach, gleich oder verschieden substituiertes Cycloalkyl mit 3 bis 6 Kohlenstoffatomen oder Cycloalkenyl mit 5 bis 7 Kohlenstoffatomen, wobei als Substituenten genannt seien: Halogen, Alkyl und Alkoxy mit jeweils 1 bis 4 Kohlenstoffatomen, Acyloxy und Acylamino mit jeweils 2 bis 5 Kohlenstoffatomen, die Oxogruppe, Phenyl, Hydroxycarbonyl und Alkoxycarbonyl mit 1 bis 4 Kohlenstoffatomen im Alkoxy teil; $R^4$ steht außerdem vorzugsweise für gegebenenfalls mit 1 oder 2 Benzol- oder Cyclohexanringen anelliertes, gegebenenfalls ein- bis fünffach, gleich oder verschieden substituiertes Phenyl, wobei als Substituenten genannt seien: Halogen, Hydroxy, Nitro, Alkyl und Alkoxy mit jeweils 1 bis 4 Kohlenstoffatomen, Halogenalkyl und Halogenalkoxy mit jeweils 1 oder 2 Kohlenstoffatomen und 2 bis 5 gleichen oder verschiedenen Halogenatomen, wie Fluor- und Chloratome, Acylamino und Acylalkylamino mit jeweils 2 bis 5 Kohlenstoffatomen im Acylteil und 1 bis 4 Kohlenstoffatomen im Alkylteil, Carboxy und Alkoxycarbonyl mit 1 bis 4 Kohlenstoffatomen im Alkoxyteil; $R^4$ steht ferner für jeweils gegebenenfalls ein- bis fünffach, gleich oder

verschieden substituiertes Phenoxy oder Phenylthio, wobei als Substituenten die oben genannten Phenylsubstituenten infrage kommen; $R^4$ steht ferner vorzugsweise für gegebenenfalls mit 1 oder 2 Benzoloder Cyclohexanringen anelliertes, gegebenenfalls ein- bis fünffach, gleich oder verschieden substituiertes 5- oder 6-gliedriges Heterocyclyl mit 1 bis 3 gleichen oder verschiedenen Heteroatomen, wie Stickstoff-, Sauerstoff- und Schwefelatome, wobei als Substituenten genannt seien: Halogen, Alkyl mit 1 bis 4 Kohlenstoffatomen, Acyl mit 2 bis 9 Kohlenstoffatomen, Phenyl, die Oxogruppe und Hydroxy; $R^4$ steht schließlich auch vorzugsweise für Alkoxycarbonyl mit 1 bis 4 Kohlenstoffatomen im Alkoxyteil, für 1-Cyanoalkoximino mit 1 oder 2 Kohlenstoffatomen im Alkoxyteil, für $-OR^5$, $-SR^5$ oder $-NR^6R^7$;

oder

$R^3$ und $R^4$ gemeinsam mit dem Stickstoffatom und gegebenenfalls dem Rest A einen gegebenenfalls ein- bis fünffach, gleich oder verschieden substituiertes und gegebenenfalls durch 1 oder 2 6-gliedrige, gesättigte oder ungesättigte Carbocyclen anellierten 5- bis 7-gliedrigen, gesättigten oder ungesättigten heterocyclischen Ring bilden, der gegebenenfalls weitere 1 oder 2 gleiche oder verschiedene Heteroatomen aus der Reihe Sauerstoff, Schwefel, Stickstoff enthält, wobei als Substituenten genannt seien: Halogen, Alkyl, Alkylthio oder Alkoxy mit jeweils 1 bis 4 Kohlenstoffatomen, Hydroxy, Mercapto, die Oxo- oder Thioxogruppe, Carboxy, Alkoxycarbonyl mit 1 bis 4 Kohlenstoffatomen im Alkoxyteil, Carbamoyl, (Di)-alkylcarbamoyl mit 1 bis 4 Kohlenstoffatomen in den jeweiligen Alkylteilen, gegebenenfalls ein-bis fünffach, gleich oder verschieden substituiertes Phenyl, wobei als Substituenten Halogen und Alkyl mit 1 bis 4 Kohlenstoffatomen infrage kommen sowie gegebenenfalls ein- bis fünffach, gleich oder verschieden substituiertes 5- oder 6-gliedriges Heterocyclyl mit 1 bis 3 gleichen oder verschiedenen Heteroatomen aus der Reihe Sauerstoff, Schwefel, Stickstoff, wobei als Substituenten Halogen und Alkyl mit 1 bis 4 Kohlenstoffatomen infrage kommen;

$R^5$ für Wasserstoff, für gegebenenfalls ein- bis fünffach, gleich oder verschieden substituiertes, geradkettiges oder verzweigtes Alkyl mit 1 bis 6 Kohlenstoffatomen, wobei folgende Substituenten infrage kommen: Halogen, $-OR^{IV}$, $-SR^{IV}$, $-COOR^{I}$, $-CONR^{II}$, CN, $-NR^{II}R^{III}$, Acyl mit 2 bis 9 Kohlenstoffatomen, jeweils gegebenenfalls einfach bis fünffach, gleich oder verschieden durch Halogen und Alkyl mit 1 bis 4 Kohlenstoffatomen substituiertes Phenyl oder Phenoxy, jeweils gegebenenfalls einbis fünffach, gleich oder verschieden durch Alkyl mit 1 bis 4 Kohlenstoffatomen substituiertes Cycloalkyl mit 3 bis 6 Kohlenstoffatomen oder Cycloalkenyl mit 5 bis 7 Kohlenstoffatomen, gegebenenfalls ein- bis fünffach, gleich oder verschieden durch Halogen und Alkyl mit 1 bis 4 Kohlenstoffatomen substituiertes 3- bis 6-gliedriges Heterocyclyl mit 1 bis 3 gleichen oder verschiedenen Heteroatomen, wie Stickstoff-, Sauerstoff- und Schwefelatomen; $R^5$ steht ferner vorzugsweise für jeweils gegebenenfalls ein- bis fünffach, gleich oder verschieden durch Alkyl mit 1 bis 4 Kohlenstoffatomen substituiertes Alkenyl oder Alkinyl mit jeweils 2 bis 6 Kohlenstoffatomen, für jeweils gegebenenfalls ein- bis fünffach, gleich oder verschieden durch Alkyl mit 1 bis 4 Kohlenstoffatomen substituiertes Cycloalkyl mit 3 bis 6 Kohlenstoffatomen oder Cycloalkenyl mit 5 bis 7 Kohlenstoffatomen;

$R^6$ für Wasserstoff oder für gegebenenfalls gleich oder verschieden, ein- oder zweifach substituiertes, geradkettiges oder verzweigtes Alkyl mit 1 bis 4 Kohlenstoffatomen, wobei als Substituenten vorzugsweise genannt seien: Halogen, Cyano, $-COOR^{I}$, $-CONR^{II}R^{III}$, $-NR^{II}R^{III}$, $-OR^{IV}$, $-S(O)_nR^{IV}$, Acyl mit 2 bis 9 Kohlenstoffatomen, gegebenenfalls ein- bis fünffach, gleich oder verschieden durch Halogen und Alkyl mit 1 bis 4 Kohlenstoffatomen substituiertes Phenyl, gegebenenfalls ein- bis fünffach, gleich oder verschieden durch Halogen, Alkyl und Alkoxy mit jeweils 1 bis 4 Kohlenstoffatomen substituiertes 5- oder 6-gliedriges Heterocyclyl mit 1 bis 3 gleichen oder verschiedenen Heteroatomen, z. B. Stickstoff, Sauerstoff und Schwefel, jeweils gegebenenfalls ein- bis fünffach, gleich oder verschieden durch Alkyl mit 1 bis 4 Kohlenstoffatomen substituiertes Cycloalkyl mit 3 bis 6 Kohlenstoffatomen oder Cycloalkenyl mit 5 bis 7 Kohlenstoffatomen; $R^6$ steht ferner vorzugsweise für geradkettiges oder verzweigtes Alkenyl oder Alkinyl mit jeweils 2 bis 6 Kohlenstoffatomen; für gegebenenfalls ein- bis fünffach, gleich oder verschieden durch Alkyl mit 1 bis 4 Kohlenstoffatomen substituiertes Cycloalkenyl mit 5 bis 7 Kohlenstoffatomen oder für gegebenenfalls ein- bis fünffach, gleich oder verschieden substituiertes Cyloalkyl mit 3 bis 6 Kohlenstoffatomen, wobei als Substituenten vorzugsweise genannt seien: Halogen, Alkyl und Alkoxy mit jeweils 1 bis 4 Kohlenstoffatomen, Cyano, Amino, Carbamoyl, Alkylamino, Dialkylamino, Alkylcarbamoyl und Dialkylcarbamoyl mit jeweils 1 bis 4 Kohlenstoffatomen in jedem Alkylteil, Alkoxycarbonylamino mit 1 bis 4 Kohlenstoffatomen im Alkoxyteil, Cycloalkyl und Cycloalkylalkyl mit 5 oder 6 Kohlenstoffatomen im Cycloalkylteil und 1 bis 4 Kohlenstoffatomen im geradkettigen oder verzweigten Alkylteil, gegebenenfalls ein- bis fünffach, gleich oder verschieden durch Halogen und Alkyl mit 1 bis 4 Kohlenstoffatomen aubstituiertes Phenyl oder Pyrroli don; $R^6$ steht weiterhin vorzugsweise für gegebenenfalls ein- bis fünffach, gleich oder verschieden durch Halogen, Alkyl oder Alkoxy mit jeweils 1 bis 4 Kohlenstoffatomen, Halogenalkyl oder Halogenalkoxy mit jeweils 1 oder 2 Kohlenstoffatomen und 1 bis 5 gleichen oder verschiedenen Halogenatomen substituiertes

Phenyl oder für einen gegebenenfalls ein- bis fünffach, gleich oder verschieden substituierten 5- oder 6-gliedrigen Heterocyclus mit 1 bis 3 gleichen oder verschiedenen Heteroatomen, wie insbesondere Stickstoff-, Sauerstoff- und Schwefelatomen; als Substituenten seien genannt: Halogen, Mercapto, Phenyl, geradkettiges oder verzweigtes Alkyl, Alkoxy, Alkylthio, Alkylsulfinyl und Alkylsulfonyl mit 1 bis 4 Kohlenstoffatomen je Alkylteil;

$R^7$ für die Bedeutungen von $R^6$ oder die Gruppierung $-OR^{IV}$;

oder

$R^6$ und $R^7$ gemeinsam mit dem Stickstoffatom, an das sie gebunden sind, für einen gegebenenfalls ein- bis fünffach, gleich oder verschieden substituierten mono-, bi- oder tricyclischen Heterocyclus oder Spiroheterocyclus mit ein bis drei weiteren, gleichen oder verschiedenen Heteroatomen, wie Sauerstoff-, Stickstoff- oder Schwefelatome, wobei als Substituenten genannt seien:

geradkettiges oder verzweigtes Alkyl oder Alkoxy mit jeweils 1 bis 4 Kohlenstoffatomen, Cycloalkyl mit 3 bis 6 Kohlenstoffatomen, die Hydroxy- oder die Oxo-Gruppe, geradkettiges oder verzweigtes Alkenyl mit 2 bis 4 Kohlenstoffatomen, Carboxy, Alkoxycarbonyl mit 1 bis 4 Kohlenstoffatomen im geradkettigen oder verzweigten Alkylteil, Carbamoyl, Alkyl- oder Dialkylcarbamoyl mit jeweils 1 bis 4 Kohlenstoffatomen in jedem geradkettigen oder verzweigten Alkylteil, jeweils gegebenenfalls einoder zweifach, gleich oder verschieden durch Halogen und geradkettiges oder verzweigtes Alkyl und Alkoxy mit jeweils 1 bis 4 Kohlenstoffatomen substituiertes Phenyl oder Phenylalkyl mit 1 bis 4 Kohlenstoffatomen im geradkettigen oder verzweigten Alkylteil;

$R^I$ für Wasserstoff oder geradkettiges oder verzweigtes Alkyl mit 1 bis 6 Kohlenstoffatomen,

$R^{II}$ und $R^{III}$ gleich oder verschieden sind und für Wasserstoff, geradkettiges oder verzweigtes Alkyl mit 1 bis 6 Kohlenstoffatomen, gegebenenfalls einbis fünffach, gleich oder verschieden substituiertes Phenylalkyl mit 1 bis 4 Kohlenstoffatomen im geradkettigen oder verzweigten Alkylteil, wobei als Phenylsubstituenten genannt seien: Halogen, Alkyl oder Alkoxy mit jeweils 1 bis 4 Kohlenstoffatomen, Halogenalkyl oder Halogenalkoxy mit jeweils 1 oder 2 Kohlenstoffatomen und 2 bis 5 gleichen oder verschiedenen Halogenatomen; $R^{II}$ steht ferner für Alkoxycarbonylalkyl, Carbamoylalkyl, Alkylcarb amoylalkyl oder Dialkylcarbamoylalkyl mit jeweils 1 bis 4 Kohlenstoffatomen in jedem Alkylteil oder für gegebenenfalls ein- bis fünffach, gleich oder verschieden durch geradkettiges oder verzweigtes Alkyl mit 1 bis 4 Kohlenstoffatomen substituiertes Cycloalkyl mit 3 bis 6 Kohlenstoffatomen stehen;

$R^{IV}$ für Wasserstoff, geradkettiges oder verzweigtes Alkyl mit 1 bis 6 Kohlenstoffatomen, gegebenenfalls einbis fünffach, gleich oder verschieden substituiertes Phenylalkyl mit 1 bis 4 Kohlenstoffatomen im geradkettigen oder verzweigten Alkylteil, wobei als Phenylsubstituenten vorzugsweise die bei $R^{II}$ genannten Phenylsubstituenten infrage kommen; $R^{IV}$ steht ferner für Acyl mit 2 bis 9 Kohlenstoffatomen;

$R^V$ für Wasserstoff, geradkettiges oder verzweigtes Alkyl mit 1 bis 6 Kohlenstoffatomen oder für gegebenenfalls ein- bis fünffach, gleich oder verschieden substituiertes Phenylalkyl mit 1 bis 4 Kohlenstoffatomen im geradkettigen oder verzweigten Alkylteil, wobei als Phenylsubstituenten die bei $R^{II}$ genannten Phenylsubstituenten infrage kommen.

Besonders bevorzugt sind substituierte Aminale der allgemeinen Formel (I), bei denen

A für -CO-, -CS- oder $-SO_2-$ steht,

n für 0 oder 1 steht,

$R^1$ für gegebenenfalls ein- bis dreifach, gleich oder verschieden substituiertes, geradkettiges oder verzweigtes Alkyl mit 1 bis 4 Kohlenstoffatomen steht, wobei folgende Substituenten infrage kommen: Fluor, Chlor, Brom, Iod, Cyano, $-COOR^I$, $-CON^{II}R^{III}$, $-OR^{IV}$, Acyl mit 2 bis 9 Kohlenstoffatomen, gegebenenfalls ein- bis dreifach, gleich oder verschieden durch Fluor, Chlor, Methyl und Methoxy substituiertes Phenyl, jeweils gegebenenfalls ein- bis dreifach durch Methyl substituiertes Cyclopropyl oder Cyclohexyl, gegebenenfalls einbis dreifach, gleich oder verschieden durch Alkyl mit 1 oder 2 Kohlenstoffatomen substituierte Heterocyclen der Formeln

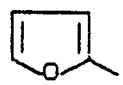 , 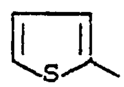 , 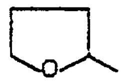 , 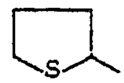 , 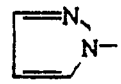

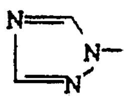 , 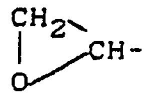 , 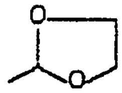 und 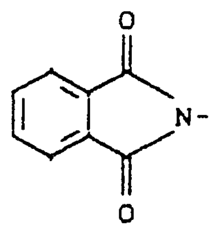 ;

$R^1$ steht ferner für gegebenenfalls ein- oder zweifach durch Methyl substituiertes Allyl oder Propargyl, für jeweils gegebenenfalls ein- bis dreifach durch Methyl substituiertes Cyclopropyl, Cyclohexyl, Cyclopentenyl oder Cyclohexenyl;

$R^2$ für Wasserstoff oder für gegebenenfalls ein- bis dreifach, gleich oder verschieden substituiertes, geradkettiges oder verzweigtes Alkyl mit 1 bis 4 Kohlenstoffatomen steht, wobei folgende Substituenten infrage kommen: Cyano, Methoxy, Ethoxy, Methylthio, Ethylthio, $-COOR^I$, Acylamino mit 2 bis 9 Kohlenstoffatomen, gegebenenfalls ein- bis dreifach, gleich oder verschieden durch Halogen, Methyl und Methoxy substituiertes Phenyl, jeweils gegebenenfalls ein- bis dreifach durch Methyl substituiertes Cyclopropyl oder Cyclohexyl sowie gegebenenfalls ein- bis dreifach, gleich oder verschieden durch Alkyl mit 1 oder 2 Kohlenstoffatomen substituierte Heterocyclen der Formeln

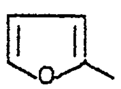 , 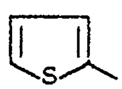 , 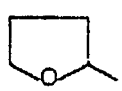 , 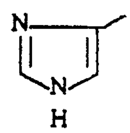 , 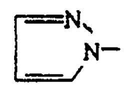

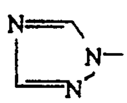 , 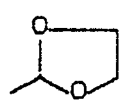 und 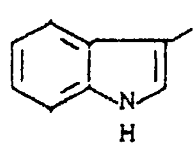 ;

$R^2$ steht ferner für jeweils gegebenenfalls durch Phenyl, das gegebenenfalls ein- bis dreifach, gleich oder verschieden durch Halogen und Methyl substituiert sein kann, substituiertes Allyl, Allenyl, Vinyl, Propargyl oder Ethinyl; $R^2$ steht außerdem für Cyclopropyl, Cyclohexyl, Cyclopentenyl oder Cyclohexenyl; $R^2$ steht weiterhin für gegebenenfalls ein- bis dreifach, gleich oder verschieden durch Halogen, Methyl und Methoxy substituiertes Phenyl, für gegebenenfalls ein- bis dreifach, gleich oder verschieden durch Alkyl mit 1 oder 2 Kohlenstoffatomen substituierte Heterocyclen der Formeln

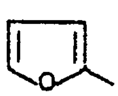 , 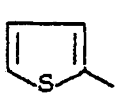 , 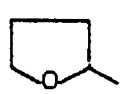 , 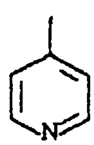 , 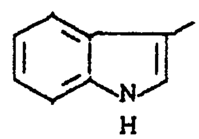 ,

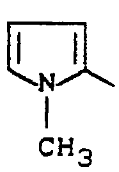 , 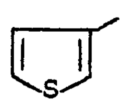 ;

$R^3$ für gegebenenfalls ein- bis dreifach, gleich oder verschieden substituiertes, geradkettiges oder verzweigtes Alkyl mit 1 bis 4 Kohlenstoffatomen steht, wobei folgende Substituenten infrage kommen: Fluor, Chlor, Brom, Iod, Cyano, $COOR^{I}$, $-CONR^{II}R^{III}$, $-OR^{IV}$, die Oxo- oder Thioxogruppe, gegebenenfalls ein- bis dreifach, gleich oder verschieden, durch Fluor, Chlor, Methyl und Methoxy substituiertes Phenyl; weitere Alkylsubstituenten sind jeweils gegebenenfalls ein- bis dreifach, gleich oder verschieden durch Halogen oder Methyl substituiertes Cyclopropyl, Cyclopentyl oder Cyclohexyl, gegebenenfalls ein- bis dreifach, gleich oder verschieden, durch Chlor, Methyl, Methoxy, Hydroxy, Methylthio, die Oxo- oder Thioxogruppe, Carboxy, Methoxycarbonyl, Ethoxycarbonyl sowie Carbamoyl substituiertes Heterocyclyl der Formeln:

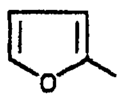 , 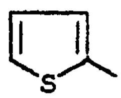 , 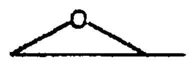 , 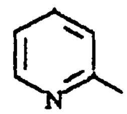 , 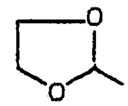

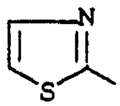 , 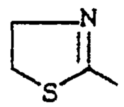 , 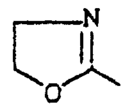 , 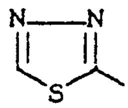 , 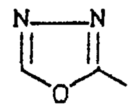

$R^3$ steht ferner für gegebenenfalls ein- bis zweifach durch Halogen oder Methyl substituiertes Allyl oder Propargyl; für jeweils gegebenenfalls ein- bis dreifach durch Methyl substituiertes Cyclopropyl, Cyclopentyl oder Cyclohexyl; für gegebenenfalls ein- bis dreifach gleich oder verschieden durch Fluor, Chlor, Methyl oder Methoxy substituiertes Phenyl; sowie für gegebenenfalls ein- bis dreifach, gleich oder verschieden, durch Fluor, Chlor, Methyl, Methoxy, Methylthio, Hydroxy, Mercapto, die Oxo- oder Thioxogruppe, durch Carboxy, Methoxycarbonyl, Ethoxycarbonyl sowie durch Carbamoyl substituierte Heterocyclen der Formeln

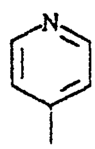 , 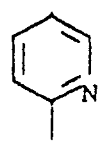 , 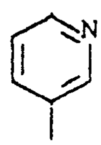 , 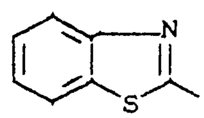 , 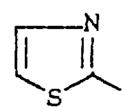

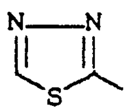 , 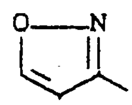 , 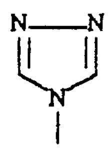 , 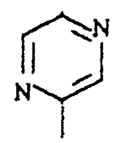 , 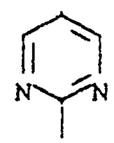 ,

und weiterhin für $OR^{V}$;
außerdem steht
$R^3$ für Wasserstoff
für den Fall daß entweder n = 1 ist und A für $-SO_2$-steht oder n = 1 ist und $R^4$ für Cyanmethoximino oder Phenylthio steht,
$R^4$ für Wasserstoff oder für gegebenenfalls ein- bis dreifach, gleich oder verschieden substituiertes, geradkettiges oder verzweigtes Alkyl mit 1 bis 4 Kohlenstoffatomen steht, wobei folgende Substituenten infrage kommen: Fluor, Chlor, Brom, Iod, Cyano, Alkoxy, Alkylthio, Alkylsulfinyl und Alkylsulfonyl mit jeweils 1 oder 2 Kohlenstoffatomen, Acyloxy und Acylamino mit jeweils 2 bis 9 Kohlenstoffatomen, $-COOR^{I}$, gegebenenfalls ein- bis dreifach, gleich oder verschieden durch Halogen und Methyl substituiertes Phenyl, Cyclopropyl, Cyclopentyl oder Cyclohexyl, gegebenenfalls ein- bis dreifach, gleich oder verschieden durch Alkyl mit 1 oder 2 Kohlenstoffatomen substituierte Heterocyclen der Formeln

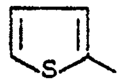 , 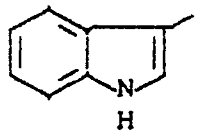 , 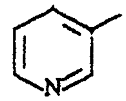 , 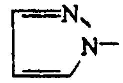 ,

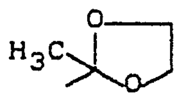 , 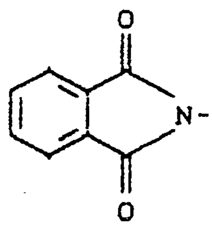 , 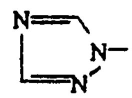 , 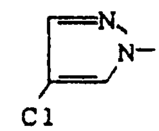 ,

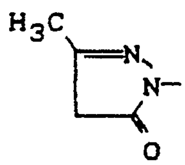 , 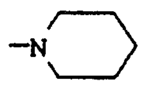 , 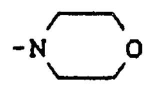 ;

$R^4$ steht ferner für jeweils gegebenenfalls durch Methyl oder Phenyl, das gegebenenfalls ein- bis dreifach, gleich oder verschieden durch Halogen und Methyl substituiert sein kann, substituiertes Vinyl, Allyl oder Ethinyl; $R^4$ steht weiterhin für jeweils gegebenenfalls mit Methylen oder Ethylen überbrücktes und/oder mit 1 oder 2 Benzol-, Cyclopentan- oder Cyclohexanringen anelliertes, jeweils gegebenenfalls ein- bis dreifach, gleich oder verschieden substituiertes Cyclopropyl, Cyclopentyl, Cyclohexyl, Cyclopentenyl oder Cyclohexenyl, wobei als Substituenten genannt seien: Halogen, Methyl, Methoxy, Acyloxy und Acylamino mit jeweils 2 bis 5 Kohlenstoffatomen, die Oxogruppe, Phenyl, Hydroxycarbonyl, Methoxy- und Ethoxycarbonyl; $R^4$ steht außerdem für gegebenenfalls mit 1 oder 2 Benzol- oder Cyclohexanringen anelliertes, gegebenenfalls ein- bis dreifach, gleich oder verschieden substituiertes Phenyl, wobei als Substituenten genannt seien: Fluor, Chlor, Hydroxy, Nitro, Methyl, Methoxy, Acylamino und HN-Alkyl-acyl-amino mit jeweils 2 bis 5 Kohlenstoffatomen im Acylteil und 1 oder 2 Kohlenstoffatomen im Alkylteil, Carboxy, Methoxy- und Ethoxycarbonyl; $R^4$ steht für jeweils gegebenenfalls ein- bis dreifach, gleich oder verschieden substituiertes Phenoxy oder Phenylthio, wobei als Substituenten die oben genannten Phenylsubstituenten infrage kommen; $R^4$ steht ferner für gegebenenfalls mit 1 oder 2 Benzol- oder Cyclohexanringen anelliertes, gegebenenfalls ein- bis dreifach, gleich oder verschieden substituiertes 5- oder 6-gliedriges Heterocyclyl mit 1 bis 3 gleichen oder verschiedenen Heteroatomen, wie Stickstoff-, Sauerstoff- und Schwefelatomen, wobei als Substituenten genannt seien: Acyl mit 2 bis 5 Kohlenstoffatomen, Chlor, Brom, Methyl, Ethyl, Phenyl, Oxo und Hydroxy; $R^4$ steht schließlich auch für Alkoxycarbonyl mit 1 oder 2 Kohlenstoffatomen im Alkoxyteil, für 1-Cyanomethoximino, $-OR^5$, $-SR^5$ oder $-NR^6R^7$;

oder

$R^3$ und $R^4$ gemeinsam für jeweils gegebenenfalls ein- bis fünffach, gleich oder verschieden, durch Methyl, Methoxy, Methylthio, Hydroxy, Mercapto, die Oxo-oder Thioxogruppe, Carboxy, Methoxycarbonyl, Ethoxycarbonyl, Carbamoyl oder Phenyl substituiertes Heterocyclyl der Formeln

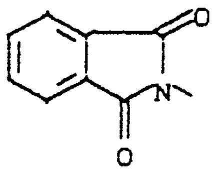 , 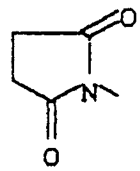 , 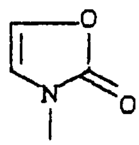 , 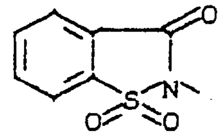 ,

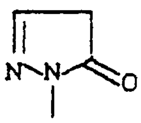 , 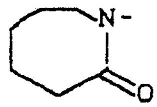 , 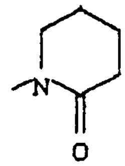 , 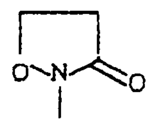 ,

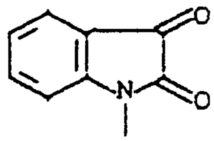 , 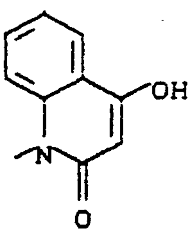 , 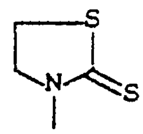 , 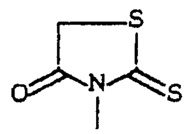 ,

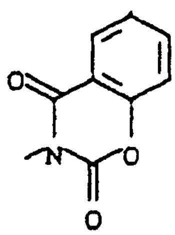 , 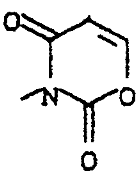 , 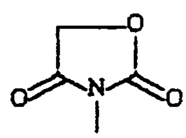 , 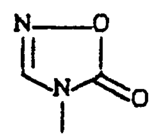 ,

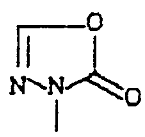 , 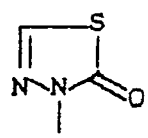 ,

vorzugsweise der Formeln

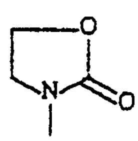 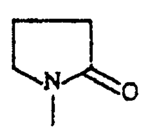 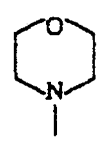

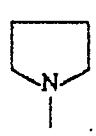 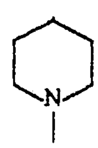 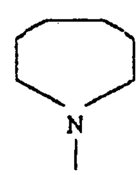

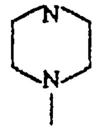

$R^5$ für Wasserstoff, für gegebenenfalls ein- bis dreifach, gleich oder verschieden substituiertes, geradkettiges oder verzweigtes Alkyl mit 1 bis 4 Kohlenstoffatomen steht, wobei folgende Substituenten infrage kommen: Fluor, Chlor, $-OR^{IV}$, $-SR^{IV}$, $-COOR^{I}$, $-CONR^{II}R^{III}$, CN, $NR^{II}R^{III}$, Alkylcarbonyl mit 1 bis 4 Kohlenstoffatomen im geradkettigen oder verzweigten Alkylteil, jeweils gegebenenfalls ein- bis dreifach, gleich oder verschieden durch Halogen und Methyl substituiertes Phenyl oder Phenoxy, jeweils gegebenenfalls ein- bis dreifach durch Methyl substituiertes Cyclopropyl, Cyclohexyl, Cyclopentenyl oder Cyclohexenyl, gegebenenfalls ein- bis dreifach, gleich oder verschieden durch Halogen, Methyl und Ethyl substituiertes 3- bis 6-gliedriges Heterocyclyl mit 1 bis 3 gleichen oder verschiedenen Heteroatomen, wie Stickstoff-, Sauerstoff- und Schwefelatomen; $R^5$ steht ferner für gegebenenfalls ein- oder zweifach durch rtethyl substituiertes Allyl oder Propargyl sowie für jeweils gegebenenfalls ein- bis dreifach durch Methyl substituiertes Cyclopropyl, Cyclohexyl, Cyclopentenyl oder Cyclohexenyl:

$R^6$ für Wasserstoff oder für gegebenenfalls ein- oder zweifach, gleich oder verschieden substituiertes geradkettiges oder verzweigtes Alkyl mit 1 bis 4 Kohlenstoffatomen steht, wobei als Substituenten vorzugsweise genannt seien: Halogen, Cyano, $-COOR^{I}$, $-CONR^{II}R^{III}$, $NR^{II}R^{III}$, $-OR^{IV}$, $-S(O)_nR^{IV}$, Acyl mit 2 bis 9 Kohlenstoffatomen, gegebenenfalls ein- bis dreifach, gleich oder verschieden durch Halogen und Alkyl mit 1 bis 4 Kohlenstoffatomen substituiertes Phenyl, gegebenenfalls ein- bis dreifach, gleich oder verschieden durch Halogen, geradkettiges oder verzweigtes Alkyl und Alkoxy mit jeweils 1 bis 4 Kohlenstoffatomen substituiertes 5- oder 6-gliedriges Heterocyclyl mit 1 bis 3 gleichen oder verschiedenen Heteroatomen, insbesondere Stickstoff, Sauerstoff und Schwefel, und jeweils gegebenenfalls ein- bis dreifach, gleich oder verschieden durch geradkettiges oder verzweigtes Alkyl mit 1 bis 4 Kohlenstoffatomen substituiertes Cycloalkyl mit 3 bis 6 Kohlenstoffatomen oder Cycloalkenyl mit 5 bis 7 Kohlenstoff atomen: $R^6$ steht ferner besonders bevorzugt für geradkettiges oder verzweigtes Alkenyl oder Alkinyl mit jeweils 2 bis 6 Kohlenstoffatomen; für gegebenenfalls ein- bis dreifach, gleich oder verschieden durch geradkettiges oder verzweigtes Alkyl mit 1 bis 4 Kohlenstoffatomen substituiertes Cycloalkenyl mit 5 bis 7 Kohlenstoffatomen oder für gegebenenfalls ein- bis dreifach, gleich oder verschieden substituiertes Cycloalkyl mit 3 bis 6 Kohlenstoffatomen, wobei als Substituenten vorzugsweise genannt seien: Halogen, geradkettiges oder verzweigtes Alkyl und Alkoxy mit jeweils 1 bis 4 Kohlenstoffatomen, Cyano, Amino, Carbamoyl, jeweils geradkettiges oder verzweigtes Alkylamino, Dialkylamino, Alkylcarbamoyl und Dialkylcarbamoyl mit jeweils 1 bis 4 Kohlenstoffatomen in jedem Alkylteil, Alkoxycarbonylamino mit 1 bis 4 Kohlenstoffatomen im geradkettigen oder verzweigten Alkoxyteil, Cycloalkyl und Cycloalkylalkyl mit jeweils 5 oder 6 Kohlenstoffatomen im Cycloalkylteil und 1 bis 4 Kohlenstoffatomen im geradkettigen oder verzweigten Alkylteil, gegebenenfalls ein- bis dreifach, gleich oder verschieden durch Halogen und Alkyl mit 1 bis 4 Kohlenstoffatomen substituiertes Phenyl oder Pyrrolidon; $R^6$ steht weiterhin besonders bevorzugt für gegebenenfalls ein- bis dreifach, gleich oder verschieden durch Halogen, geradkettiges oder verzweigtes Alkyl oder Alkoxy mit jeweils 1 bis 4 Kohlenstoffatomen, Halogenalkyl oder Halogenalkoxy mit jeweils 1 oder 2 Kohlenstoffatomen und 1 bis 5 gleichen oder verschiedenen Halogenatomen substituiertes Phenyl oder für einen gegebenenfalls ein- bis dreifach, gleich oder verschieden substituierten 5- oder 6-gliedrigen Heterocyclus mit 1 bis 3 gleichen oder verschiedenen Heteroatomen, wie insbesondere Stickstoff, Sauerstoff oder Schwefel; als Substituenten für die Heterocyclen seien genannt: Halogen, Mercapto, Phenyl, geradkettiges oder verzweigtes Alkyl, Alkoxy, Alkylthio, Alkylsulfinyl und Alkylsulfonyl mit 1 bis 4 Kohlenstoffatomen je Alkylteil;

$R^7$ für die Bedeutungen von $R^6$ oder die Gruppierung $-OR^{IV}$ steht,

oder

$R^6$ und $R^7$ gemeinsam mit dem Stickstoffatom, an das sie gebunden sind, für einen gegebenenfalls einbis fünffach, gleich oder verschieden substituierten mono-, bi- oder tricyclischen Heterocyclus oder Spiroheterocyclus stehen, wobei als Heterocyclen genannt seien:

Oxazolidin, Pyrrolidin, Imidazolidin, Piperidin, Piperazin, Morpholin, Thiomorpholin, 1,3-Oxazan oder 1,3-Diazan; diese Heterocyclen können jeweils gegebenenfalls mit 1 oder 2 Benzol- oder Cyclohexanringen anelliert sein oder gegebenenfalls mit Methylen oder Ethylen überbrückt sein.

Als Substituenten für alle Heterosysteme seien genannt:

geradkettiges oder verzweigtes Alkyl oder Cycloalkyl mit 3 bis 6 Kohlenstoffatomen, die Hydroxy- oder die Oxo-Gruppe, geradkettiges oder verzweigtes Alkenyl mit 2 bis 4 Kohlenstoffatomen, Carboxy, Alkoxycarbonyl mit 1 bis 4 Kohlenstoffatomen im geradkettigen oder verzweigten Alkylteil, Carbamoyl, Alkyl- oder Dialkylcarbamoyl mit jeweils 1 bis 4 Kohlenstoffatomen in jedem Alkylteil, ferner jeweils gegebenenfals einoder zweifach, gleich oder verschieden durch Halogen und geradkettiges oder verzweigtes Alkyl und Alkoxy mit jeweils 1 bis 4 Kohlenstof fatomen substituiertes Phenyl oder Phenylalkyl mit 1 bis 4 Kohlenstoffatomen im geradkettigen oder verzweigten Alkylteil,

$R^{I}$ für Wasserstoff oder geradkettiges oder verzweigtes Alkyl mit 1 bis 4 Kohlenstoffatomen steht,

$R^{II}$ und $R^{III}$ gleich oder verschieden sind und für Wasserstoff, geradkettiges oder verzweigtes Alkyl mit 1 bis

4 Kohlenstoffatomen, jeweils gegebenenfalls ein- bis dreifach, gleich oder verschieden substituiertes Benzyl oder Phenethyl steht, wobei als Phenylsubstituenten jeweils genannt seien: Fluor, Chlor, Methyl, Methoxy und Trifluormethyl; $R^{II}$ ferner für Alkoxycarbonylalkyl mit 1 bis 4 Kohlenstoffatomen im Alkoxyteil und 1 oder 2 Kohlenstoffatomen im Alkylteil, Carbamoylalkyl mit 1 oder 2 Kohlenstoffatomen im Alkylteil, Alkylcarbamoylalkyl oder Dialkylcarbamoylalkyl mit jeweils 1 oder 2 Kohlenstoffatomen in jedem Alkylteil, oder für gegebenenfalls ein- bis dreifach, gleich oder verschieden durch Methyl und Ethyl substituiertes Cycloalkyl mit 3 bis 6 Kohlenstoffatomen stehen,

$R^{IV}$ für Wasserstoff, geradkettiges oder verzweigtes Alkyl mit 1 bis 4 Kohlenstoffatomen, jeweils gegebenenfalls ein- bis dreifach, gleich oder verschieden substituiertes Benzyl oder Phenethyl steht, wobei als Phenylsubstituenten jeweils genannt seien: Fluor, Chlor, Methyl, Methoxy und Trifluormethyl; $R^{IV}$ steht ferner für Acyl mit 2 bis 9 Kohlenstoffatomen;

$R^{V}$ für Wasserstoff, geradkettiges oder verzweigtes Alkyl mit 1 bis 4 Kohlenstoffatomen oder für jeweils gegebenenfalls ein- bis dreifach gleich oder verschieden substituiertes Benzyl oder Phenethyl steht, wobei als Phenylsubstituenten die bei $R^{II}$ genannten Phenylsubstituenten infrage kommen.

Als Beispiele für erfindungsgemäße Verbindungen seien außer den bei den Herstellungsbeispielen genannten Verbindungen die in der folgenden Tabelle 1 aufgeführten Stoffe der Formel (I) genannt:

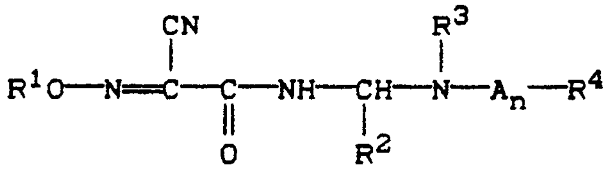

(I)

Tabelle 1

| $R^1$ | $R^2$ | 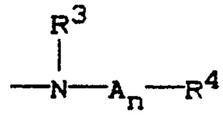 |
|---|---|---|
| $CH_3$ | H | 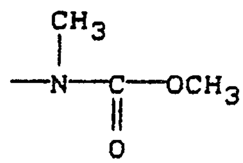 |
| $CH_3$ | H | 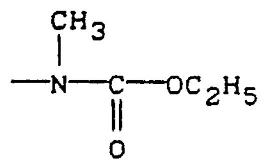 |
| $CH_3$ | H | 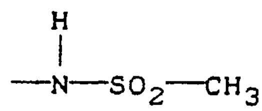 |
| $CH_3$ | H | 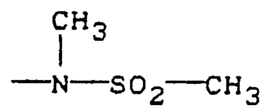 |
| $CH_3$ | H | 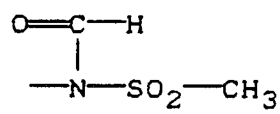 |
| $CH_3$ | H | 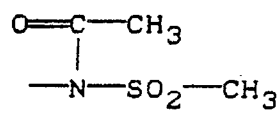 |
| $CH_3$ | H | 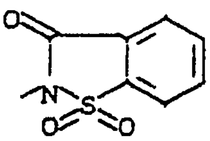 |
| $CH_3$ | H | 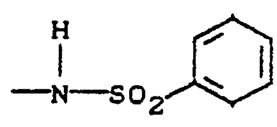 |
| $CH_3$ | H | 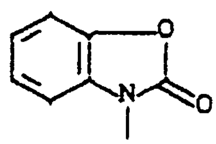 |

Tabelle 1 - Fortsetzung

| $R^1$ | $R^2$ | 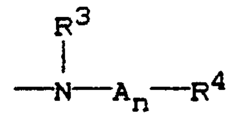 |
|---|---|---|
| $CH_3$ | H | 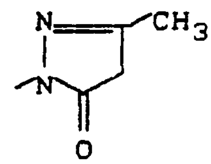 |
| $CH_3$ | H | 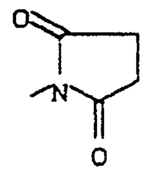 |
| $CH_3$ | H | 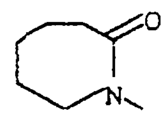 |
| $CH_3$ | H | 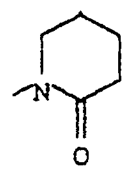 |
| $CH_3$ | H | 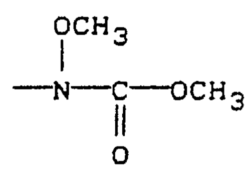 |
| $CH_3$ | H | 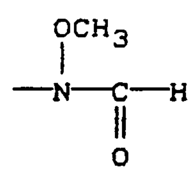 |
| $CH_3$ | H | 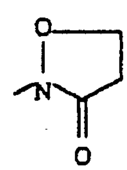 |

Tabelle 1 - Fortsetzung

| $R^1$ | $R^2$ | 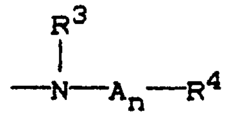 |
|---|---|---|
| $CH_3$ | H | 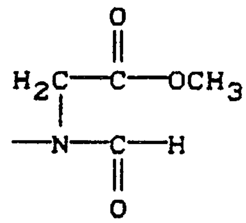 |
| $CH_3$ | H | 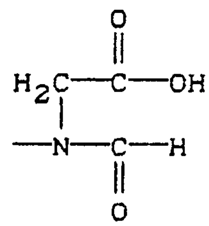 |
| $CH_3$ | H | 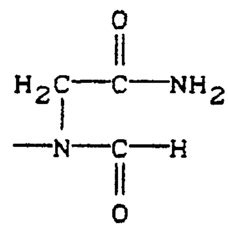 |
| $CH_3$ | H | 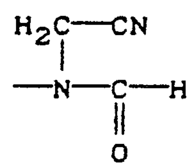 |
| $CH_3$ | H | 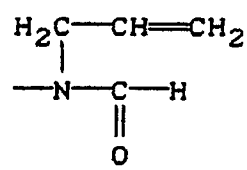 |
| $CH_3$ | H | 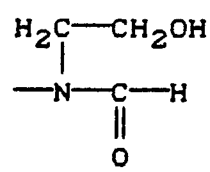 |

Tabelle 1 - Fortsetzung

| $R^1$ | $R^2$ | 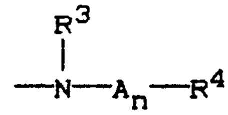 |
|---|---|---|
| $CH_3$ | H | 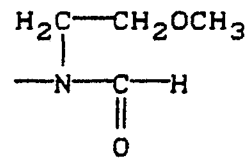 |
| $CH_3$ | H | 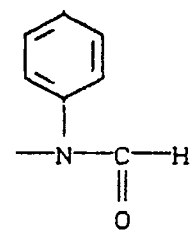 |
| $CH_3$ | H | 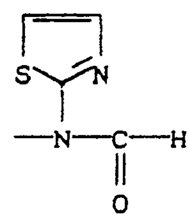 |
| $CH_3$ | H | 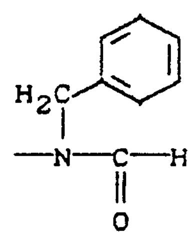 |
| $CH_3$ | H | 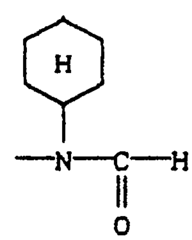 |

<u>Tabelle 1</u> - Fortsetzung

| $R^1$ | $R^2$ | 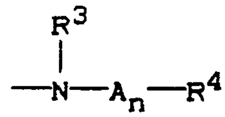 |
|---|---|---|
| $CH_3$ | H | 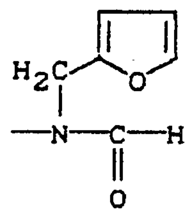 |
| $CH_3$ | H | 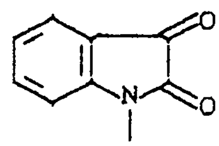 |
| $CH_3$ | H | 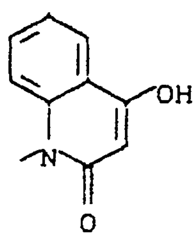 |
| $CH_3$ | H | 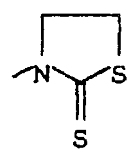 |
| $CH_3$ | H | 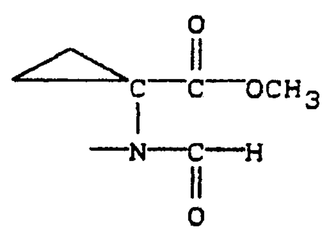 |
| $CH_3$ | H | 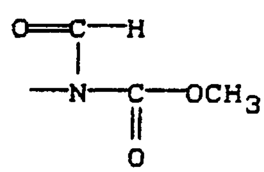 |

Tabelle 1 - Fortsetzung

| $R^1$ | $R^2$ | 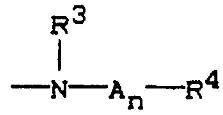 |
|---|---|---|
| $CH_3$ | H | 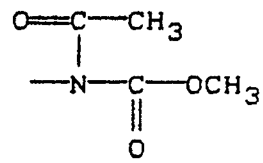 |
| $CH_3$ | H | 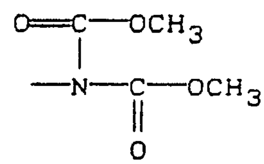 |
| $CH_3$ | H | 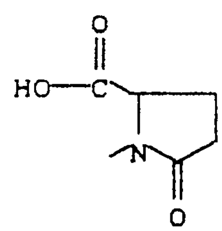 |
| $CH_3$ | H | 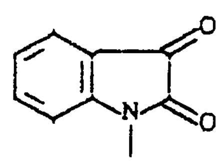 |
| $CH_3$ | H | 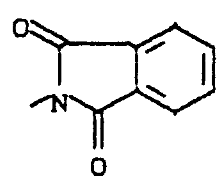 |

Tabelle 1 - Fortsetzung

| $R^1$ | $R^2$ | 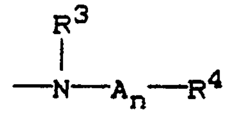 |
|---|---|---|
| $CH_3$ | H | 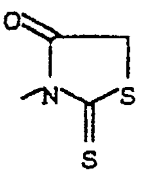 |
| $CH_3$ | H | 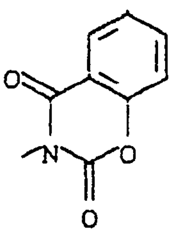 |
| $CH_3$ | H | 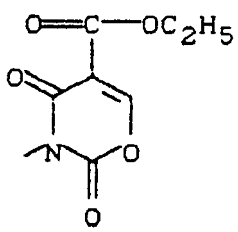 |
| $CH_3$ | H | 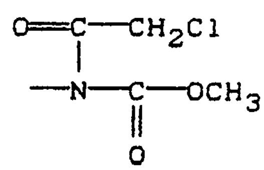 |
| $CH_3$ | H | 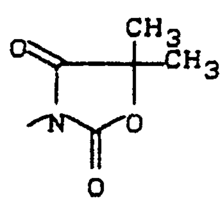 |

Tabelle 1 - Fortsetzung

| $R^1$ | $R^2$ | 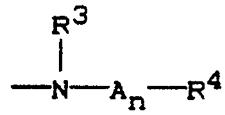 |
|---|---|---|
| $CH_3$ | H | 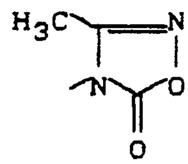 |
| $CH_3$ | H | 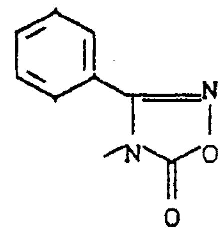 |
| $CH_3$ | H | 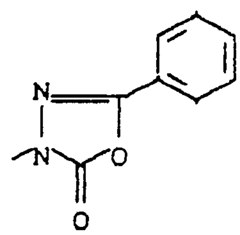 |
| $CH_3$ | $CH_3$ | 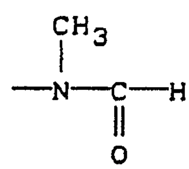 |
| 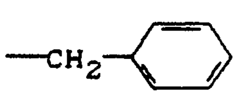 | H | 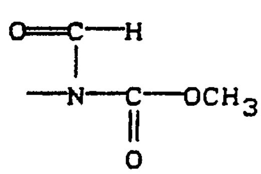 |

Tabelle 1 - Fortsetzung

| $R^1$ | $R^2$ | 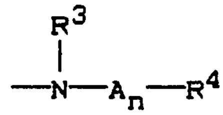 |
|---|---|---|
| $CH_3$ | 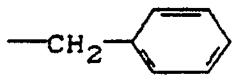 | 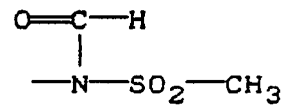 |
| $CH_3$ | 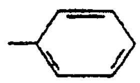 | 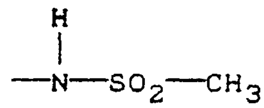 |
| $-CH_2-C\equiv N$ | H | 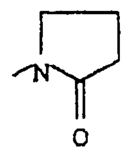 |
| 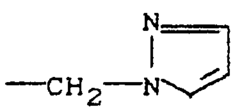 | H | 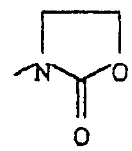 |
| $CH_3$ | 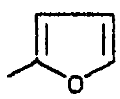 | 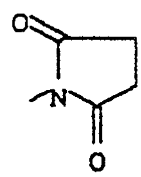 |
| $-CH_2-CH=CH_2$ | H | 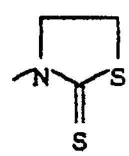 |

Tabelle 1 - Fortsetzung

| $R^1$ | $R^2$ | 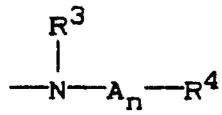 |
|---|---|---|
| $CH_3$ | H | 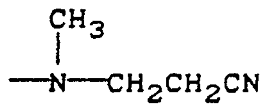 |
| $CH_3$ | H | 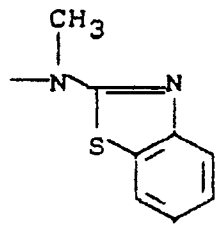 |
| $CH_3$ | H | 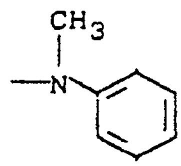 |
| $CH_3$ | H | 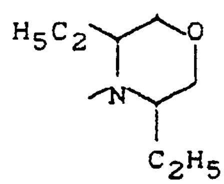 |

Verwendet man beispielsweise (E)-2-Cyano-2-methoximinoacetylchloride und N-Aminomethylphthalimid-hydrochlorid als Ausgangsstoffe, Triethylamin als Base und 4-Dimethylaminopyridin als Katalysator, so läßt sich der Reaktionsablauf des erfindungsgemäßen Verfahrens (a) durch das folgende Formelschema darstellen:

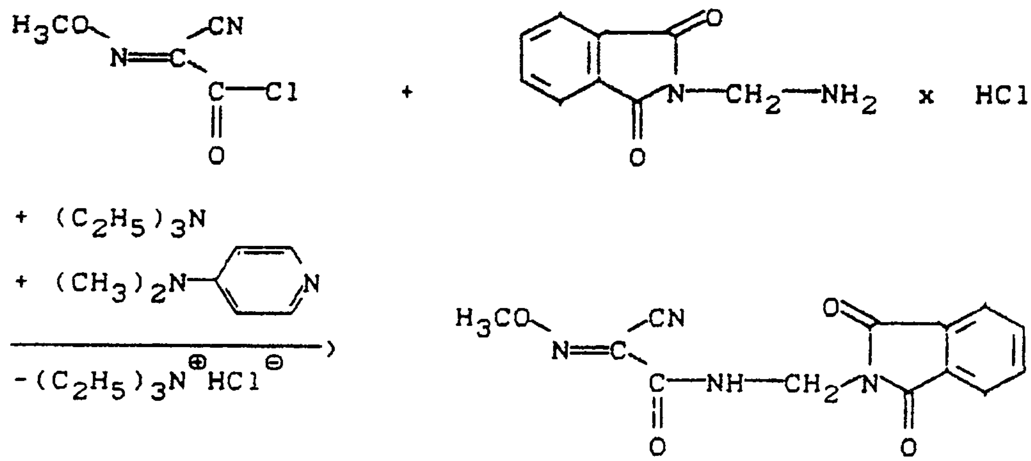


Verwendet man beispielsweise (E)-2-Cyano-2-methoximinoacetamid und N-Hydroxymethyl-2-pyrrolidon als Ausgangsstoffe und Schwefelsäure als Säure, so läßt sich der Reaktionsablauf des erfindungsgemäßen Verfahrens (b) durch das folgende Formelschema darstellen:

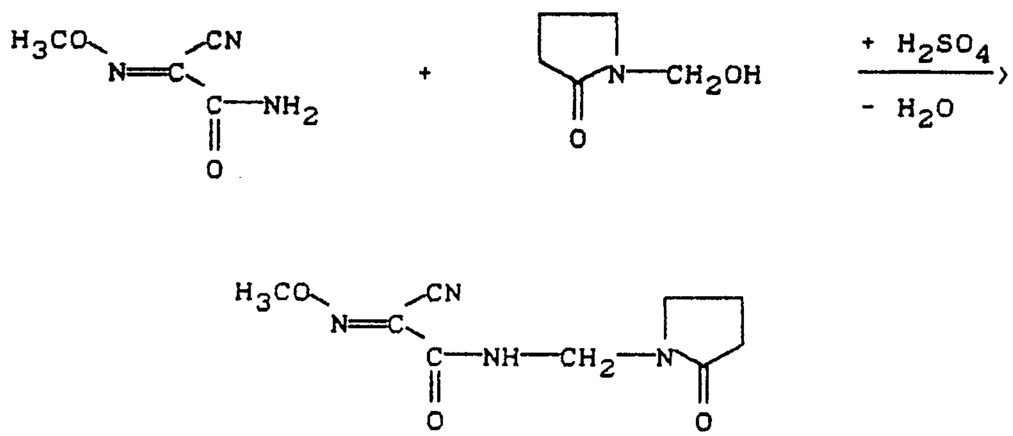

Verwendet man beispielsweise (E)-2-Cyano-2-methoximinoacetamid und N-Chlormethyl-N-methoxycarbonyl-methylamin als Ausgangsstoffe und Triethylamin als Base, so läßt sich der Reaktionsablauf des erfindungsgemäßen Verfahrens (c) durch das folgende Formelschema darstellen:

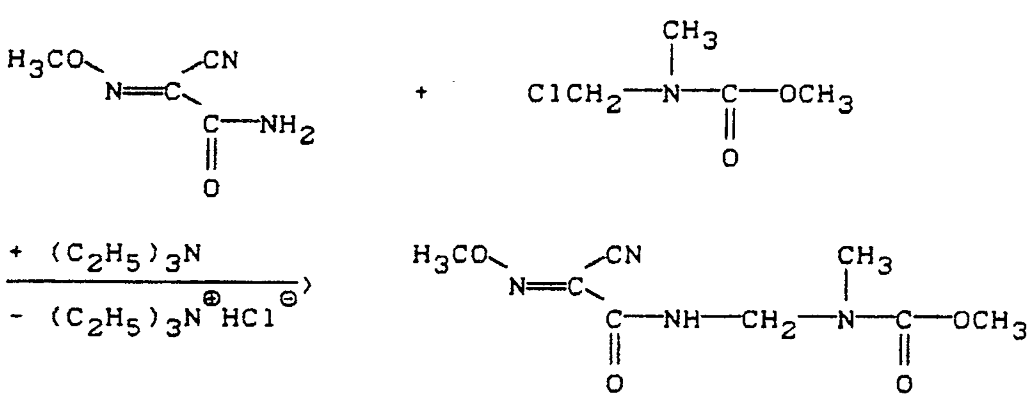


Verwendet man beispielsweise (E)-2-Cyano-2-methoximinoacetamid, Dimethylamin und Formaldehyd als Ausgangsstoffe, so läßt sich der Reaktionsablauf des erfindungsgemäßen Verfahrens (d) durch das folgende Formelschema darstellen:

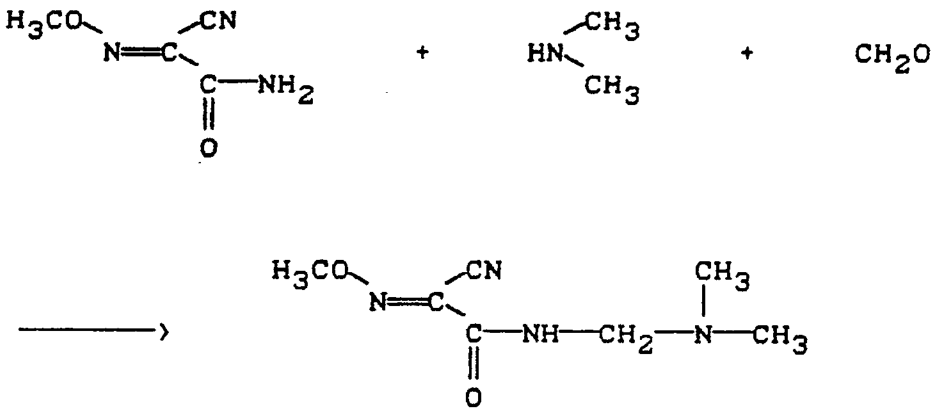

Verwendet man beispielsweise (E)-N-Aminomethyl-2-cyano-2-methoxyiminoacetamid-hydrochlorid als Ausgangsstoff, Chlorameisensäurephenylester als Acylierungsreagenz und Triethylamin als Base sowie 4-Dimethylaminopyridin als Katalysator, so läßt sich der Reaktionsablauf des erfindungsgemäßen Verfahrens (e) durch das folgende Formelschema darstellen:

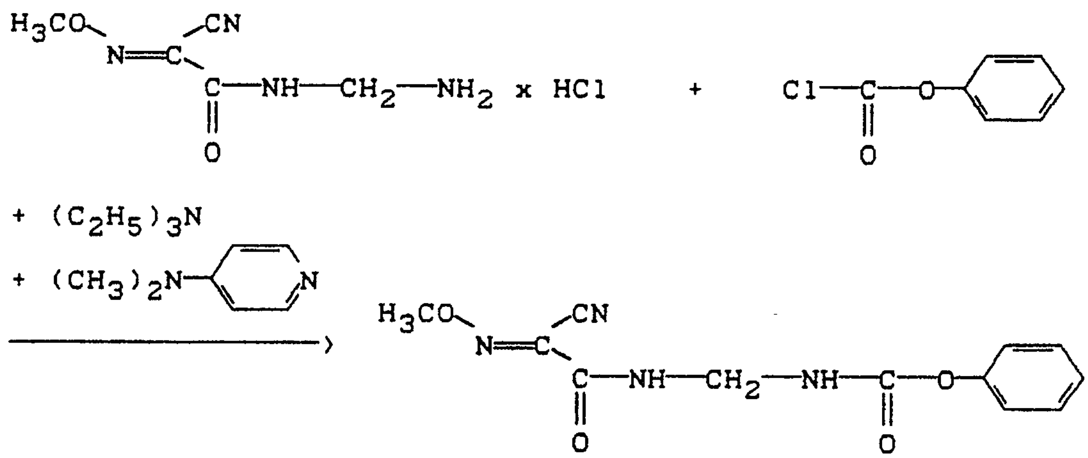


Die beim erfindungsgemäßen Verfahren (a) zur Herstellung von Verbindungen der Formel (I) als Ausgangsstoffe zu verwendenden 2-Cyano-2-oximinocarbonyl-Verbindungen sind durch die Formel (II) allgemein definiert.

In Formel (II) hat $R^1$ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^1$ angegeben wurden.

Die 2-Cyano-2-oximinocarbonyl-Verbindungen der Formel (II) sind bekannt und/oder können nach an sich bekannten Verfahren hergestellt werden (vgl. z.B. DE-OS 37 28 277 /EP 304 758).

Die beim erfindungsgemäßen Verfahren (a) zur Herstellung von Verbindungen der Formel (I) außerdem als Ausgangsstoffe zu verwendenden Amino-Verbindungen sind durch die Formel (III) allgemein definiert.

In Formel (III) haben $R^2$, $R^3$, $R^4$, A und n vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^2$, $R^3$, $R^4$, A und n angegeben wurden.

HY steht vorzugsweise für das Äquivalent einer Mineralsäure, wie z.B. Chlorwasserstoffsäure oder einer Carbonsäure, wie z.B. Oxalsäure.

Die Amino-Verbindungen der Formel (III) sind teilweise bekannt (vgl. z.B. J. Heterocycl. Chem 16, 339 (1979) oder können nach an sich bekannten Verfahren hergestellt werden, indem man Halogenverbindungen der Formel (VI)

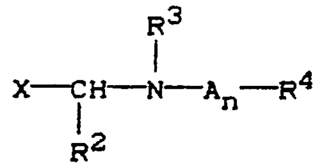

(VI)

in welcher
$R^2$, $R^3$, $R^4$, A und n die oben angegebene Bedeutung haben,
und
X für Chlor oder Brom steht,
mit Urotropin gegebenenfalls in Gegenwart eines Verdünnungsmittels, wie beispielsweise Chloroform, bei Temperaturen zwischen 0° C und 100° C umsetzt und das erhaltene Zwischenprodukt der Formel (X)

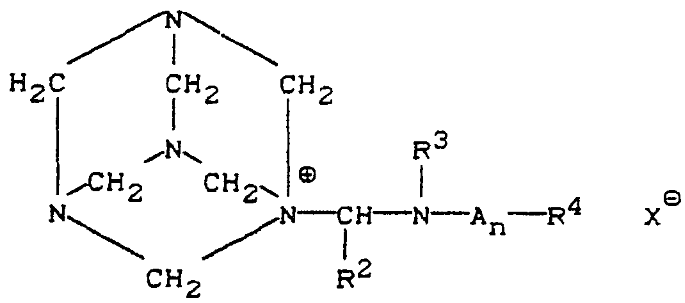

(X)

in welcher
$R^2$, $R^3$, $R^4$, A und n die oben angegebene Bedeutung haben,
und
X für Chlor oder Brom steht,
mit einer Mineralsäure wie beispielsweise Chlorwasserstoffsäure hydrolysiert.

Die Halogenverbindungen der Formel (VI)

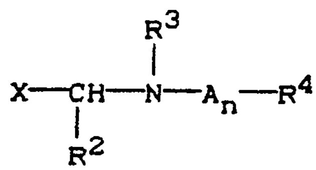

(VI)

sind teilweise bekannt (vgl. z.B. DE-OS 21 19 518) oder können hergestellt werden, indem man beispielsweise Verbindungen der Formel (XI)

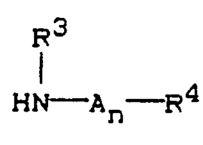

(XI)

in welcher
$R^3$, $R^4$, A und n die oben angegebene Bedeutung haben,
durch Umsetzung mit einem Aldehyd der Formel (XII)
$R^2CHO$ (XII)
in welcher
$R^2$ die oben angegebene Bedeutung hat,
und einem Halogenierungsreagenz wie beispielsweise Thionylchlorid, gegebenenfalls in Gegenwart eines Verdünnungsmittels, wie beispielsweise Toluol oder Chloroform, bei Temperaturen von 0° C bis 150° C in die Halogenverbindungen der Formel (VI) überführt.

Die Verbindungen der Formel (XI) sind bekannt und/oder können nach an sich bekannten Verfahren hergestellt werden (vgl. CA 107, 156950y). Die Aldehyde der Formel (XII) sind allgemein bekannte Synthesechemikalien.

Die bei den erfindungsgemäßen Verfahren (b), (c) und (d) zur Herstellung von Verbindungen der Formel (I) als Ausgangsstoffe zu verwendenden 2-Cyano-2-oximinoacetamide sind durch die Formel (IV) allgemein definiert.

In Formel (IV) hat $R^1$ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^1$ angegeben wurden.

Die 2-Cyano-2-oximinoacetamide der Formel (IV) sind bekannt und/oder können in Analogie zu bekannten Verfahren hergestellt werden (vgl. z.B. Chem. Ber. 54, 1342 (1921); DE-OS 26 23 847; DE-OS 26 57 145; DE-OS 37 02 283).

Die beim erfindungsgemäßen Verfahren (b) zur Herstellung von Verbindungen der Formel (I) als Ausgangsstoffe zu verwendenden Verbindungen sind durch die Formel (V) allgemein definiert.

In Formel (V) haben $R^2$, $R^3$, $R^4$, A und n vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^2$, $R^3$, $R^4$, A und n angegeben wurden.

$R^8$ steht vorzugsweise für Wasserstoff, Methyl oder Acetyl.

Die Verbindungen der Formel (V) sind bekannt (vgl. z.B. Beilstein 21 4. Ergänzungswerk; Tetrahedron Lett. 27, 3525 (1986)) und/oder können nach bekannten Verfahren hergestellt werden, indem man beispielsweise Verbindungen der Formel (XI)

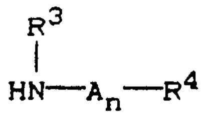

(XI)

in weloher

$R^3$, $R^4$, A und n die oben angegebene Bedeutung haben,

mit Aldehyden der Formel (XII) gegebenenfalls in Gegenwart eines Verdünnungsmittels wie Ethanol oder Essigester bei Temperaturen von 0° C bis 100° C zu den Hydroxyverbindungen der Formel (Va)

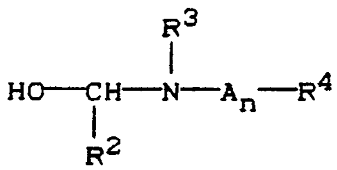

(Va)

in welcher

$R^2$, $R^3$, $R^4$, A und n die oben angegebene Bedeutung haben,

umsetzt und in einer Folgereaktion durch Acetylierung mit Acetanhydrid oder Acetylchlorid oder durch Veresterung mit Methanol in Gegenwart von Schwefelsäure oder Chlorwasserstoff die Hydroxygruppe derivatisiert.

Die beim erfindungsgemäßen Verfahren (c) zur Herstellung von Verbindungen der Formel (I) außerdem als Ausgangsstoffe zu verwendenden Halogen-Verbindungen sind durch die Formel (VI) allgemein definierte.

In Formel (VI) haben $R^2$, $R^3$, $R^4$, A und n vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^2$, $R^3$, $R^4$, A und n angegeben wurden.

X steht vorzugsweise für Chlor oder Brom.

Die Halogen-Verbindungen der Formel (VI) sind bekannt und/oder können nach bekannten Verfahren hergestellt werden (vgl. Verfahren (a)).

Die beim erfindungsgemäßen Verfahren (d) zur Herstellung von Verbindungen der Formel (I) außerdem als Ausgangsstoffe zu verwendenden Amine sind durch die Formel (VII) allgemein definiert.

In Formel (VII) haben $R^3$ und $R^4$ vorzugsweise bzw. insbesondere diejenigen Bedeutungen, die bereits oben im Zusammenhang mit der Beschreibung der erfindungsgemäßen Verbindungen der Formel (I)

vorzugsweise bzw. als insbesondere bevorzugt für $R^3$ und $R^4$ angegeben wurden.

Amine der Formel (VII) sind allgemein bekannte Verbindungen der organisohen Chemie.

Die beim erfindungsgemäßen Verfahren (e) zur Herstellung von Verbindungen der Formel (I) als Ausgangsstoffe zu verwendenden N-(2-Cyano-2-oximinino-acetyl)-aminale sind durch die Formel (VIII) allgemein definiert.

In Formel (VIII) haben $R^1$ und $R^2$ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^2$ und $R^2$ angegeben wurden.

HY steht für das Äquivalent einer Mineralsäure wie beispielsweise Chlorwasserstoffsäure oder einer Carbonsäure wie beispielsweise Oxalsäure.

Die N-(2-Cyano-2-oximino-acetyl)-aminale der Formel (VIII) sind bekannt und/oder können nach bekannten Verfahren hergestellt werden (vgl. DE-OS 37 28 277 /EP 304 758).

Die beim erfindungsgemäßen Verfahren (e) zur Herstellung von Verbindungen der Formel (I) als Ausgangsstoffe zu verwendenden Acylierungsreagenzien sind durch die Formel (IX) allgemein definiert.

In Formel (IX) haben $R^4$ und A vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^4$ und A angegeben wurden und
Q steht vorzugsweise für eine Abgangsgruppe.

Hierzu gehören vorzugsweise Chlor, Brom, Alkoxy und Alkylthio mit jeweils 1 bis 4 Kohlenstoffatomen, Carboxymethoxy, Carboxymethylthio, die Gruppierungen -O-CO-$R^4$, -O-CO-O$R^5$, -O$R^5$ und S$R^5$. Dabei haben $R^4$ und $R^5$ vorzugsweise die Bedeutungen, die bereits im Zusammenhang mit der Beschreibung der erfindungsgemäßen Stoffe der Formel (I) für diese Substituenten vorzugsweise genannt wurden.

Die Acylierungsreagenzien der Formel (IX) d.h. Carbonsäurehalogenide, Carbonsäureanhydride, Halogenameisensäureester und -thiolester, Trithiocarbonate, Pyrocarbonate, Carbamidsäurehalogenide, Carbamate, Thiolcarbamate, Dithiocarbamate, Isocyanate oder Isothiocyanate sind allgemein bekannte Verbindungen der organischen Chemie bzw. nach allgemein üblichen Methoden erhältlich.
Die erfindungsgemäßen Verfahren (a) und (e) werden vorzugsweise unter Verwendung von Verdünnungsmitteln durchgeführt.

Als Verdünnungsmittel kommen dabei inerte organische Lösungsmittel infrage. Hierzu gehören vorzugsweise aromatische Kohlenwasserstoffe, wie Toluol; halogenierte Kohlenwasserstoffe, wie Methylenchlorid oder Chloroform; Ether, wie Tetrahydrofuran oder 1,2-Dimethoxyethan; Ester, wie Essigsäureethylester; Nitrile, wie Acetonitril; Ketone, wie Aceton; tertiäre Amine, wie Pyridin; Amide, wie Dimethylformamid.

Die erfindungsgemäßen Verfahren (a) und (e) werden in Gegenwart einer Base durchgeführt. Hierbei kommen übliche organische und anorganische Basen infrage. Vorzugsweise genannt seien tertiäre Amine, wie Triethylamin oder Pyridin; Alkoholate, wie Natriummethylat und Alkalicarbonate, wie Kaliumcarbonat.

Die erfindungsgemäßen Verfahren (a) und (e) werden gegebenenfalls in Gegenwart eines Katalysators durchgeführt. Beispielsweise genannt seien tertiäre Amine, wie 4-Dimethylaminopyridin, 1,8-Diazabicyclo-[5,4,0]-undec-7-en (DBU) oder 1,4-Diazabicyclo[2,2,2]-octan (DBCO); ferner Imidazol und Dimethylformamid.

Die Reaktionstemperaturen können bei der Durchführung der erfindungsgemäßen Verfahren (a) und (e) in einem größeren Bereich variiert werden. Im allgemeinen arbeitet man bei Temperaturen von -20° C bis 120° C, vorzugsweise von 0° C bis 40° C.

Bei der Durchführung des erfindungsgemäßen Verfahrens (a) arbeitet man vorzugsweise in äquimolaren Mengen. Es ist jedoch auch möglich, eine der beiden eingesetzten Komponenten sowie die Base in einem größeren Überschuß zu verwenden.

Bei der Durchführung des erfindungsgemäßen Verfahrens (e) arbeitet man vorzugsweise in äquimolaren Mengen, wobei jedoch auch die N-(2-Cyan-2-oximino-acetyl)-aminale der Formel (VIII) oder das Acylierungsreagenz der Formel (IX) sowie die Base im Überschuß eingesetzt werden können. Die Reaktionsdurchführung und Aufarbeitung erfolgt bei den Verfahren (a) und (e) nach allgemein üblichen Methoden.

Das erfindungsgemäße Verfahren (b) wird vorzugsweise unter Verwendung von Verdünnungsmitteln durchgeführt. Als solche kommen infrage:
aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und Dichlorbenzol, Ether, wie Diethyl- und Dibutylether, Glykoldimethylether und Diglykoldimethylether, Tetrahydrofuran und Dioxan, Ester, wie Essigsäuremethylester und -ethylester, Nitrile, wie z.B. Acetonitril und Propionitril, Amide, wie z.B. Dimethylformamid, Dimethylacetamid und N-Methyl-pyrrolidon sowie Dimethylsulfoxid, Carbonsäuren, wie Essigsäure, Alkohole wie Methanol, Ethanol oder Isopropanol sowie Wasser.

Das erfindungsgemäße Verfahren (b) wird in Gegenwart von Reaktionshilfsstoffen durchgeführt. Als

solche kommen beispielsweise Mineralsäuren wie Schwefelsäure oder Chlorwasserstoffsäure infrage oder stark saure Ionenaustauscher wie Levatit S100 oder SPC 108.

Die Reaktionstemperaturen können bei dem erfindungsgemäßen Verfahren (b) in einem größeren Bereich variiert werden. Im allgemeinen arbeitet man bei Temperaturen von 0 °C bis +150 °C, vorzugsweise bei Temperaturen von +40 °C bis +100 °C.

Bei der Durchführung des erfindungsgemäßen Verfahrens (b) arbeitet man vorzugsweise in äquimolaren Mengen, es ist jedoch auch möglich, eine der beiden eingesetzten Komponenten sowie die Mineralsäure in einem größeren Überschuß zu verwenden. Verwendet man als Hilfsstoff einen Ionenaustauscher, so wird dieser in katalytischer Menge eingesetzt.

Die Reaktionsdurchführung und Aufarbeitung erfolgt nach allgemein üblichen Methoden.

Das erfindungsgemäße Verfahren (c) wird vorzugsweise unter Verwendung von Verdünnungsmitteln durchgeführt. Als solche kommen infrage:

Ether, wie Diethyl- und Dibutylether, Glykoldimethylether und Diglykoldimethylether, Tetrahydrofuran und Dioxan, Ketone, wie Aceton, Methyl-ethyl-, Methyl-isopropyl- und Methylisobutyl-keton, Ester, wie Essigsäuremethylester und -ethylester, Nitrile, wie z.B. Acetonitril und Propionitril, vorzugsweise verwendet man Acetonitril oder Essigsäureethylester.

Das erfindungsgemäße Verfahren (c) wird in Gegenwart einer Base durchgeführt. Hierbei kommen übliche organische und anorganische Basen infrage. Vorzugsweise genannt seien tertiäre Amine, wie Triethylamin oder Pyridin; Alkoholate, wie Natriummethylat und Alkalicarbonate, wie Kaliumcarbonat.

Die Reaktionstemperaturen können bei dem erfindungsgemäßen Verfahren (c) in einem größeren Bereich variiert werden. Im allgemeinen arbeitet man bei Temperaturen von 0 °C bis +160 °C, vorzugsweise bei Temperaturen von +40° C bis +1000° C.

Bei der Durchführung des erfindungsgemäßen Verfahrens (c) arbeitet man vorzugsweise in äquimolaren Mengen, es ist jedoch auch möglich, eine der beiden eingesetzten Komponenten sowie die Base in einem größeren Überschuß zu verwenden.

Die Reaktionsdurchführung und Aufarbeitung erfolgt nach allgemein üblichen Methoden.

Das erfindungsgemäße Verfahren (d) wird vorzugsweise unter Verwendung von Verdünnungsmitteln durchgeführt. Als solche kommen infrage:

Wasser, Alkohole, wie Methanol, Ethanol und t-Butanol, mit Wasser mischbare Ether, wie Glykoldimethylether und Diglykoldimethylether, Tetrahydrofuran und Dioxan, Nitrile, wie z.B. Acetonitril und Propionitril, vorzugsweise verwendet man Gemische aus Wasser und Alkohol.

Die Reaktionstemperaturen können bei dem erfindungsgemäßen Verfahren (d) in einem größeren Bereich variiert werden. Im allgemeinen arbeitet man bei Temperaturen von -20 °C bis +120 °C, vorzugsweise bei Temperaturen von 0 °C bis +100 °C.

Bei der Durchführung des erfindungsgemäßen Verfahrens (d) arbeitet man vorzugsweise in äquimolaren Mengen, es ist jedoch auch möglich, eine der drei verwendeten Komponenten in einem größeren Überschuß einzusetzen. Die Reaktionsdurchführung und Aufarbeitung erfolgt nach allgemein üblichen Methoden, die Isolierung der Reaktionsprodukte erfolgt gegebenenfalls chromatographisch.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Schädlingsbekämpfungsmittel, insbesondere als Fungizide geeignet.

Zum Beispiel werden fungizide Mittel im Pflanzenschutz eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Erkrankungen, die unter die oben aufgezähl ten Oberbegriffe fallen, genannt.

Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea (Konidienform: Drechslera, Syn: Helminthosporium);
Cochliobolus-Arten, wie beispielsweise Cochliobolus sativus (Konidienform: Drechslera, Syn: Helminthosporium);
Uromyces-Arten, wie beispielsweise Uromyces appendiculatus;
Puccinia-Arten, wie beispielsweise Puccinia recondita; Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten wie beispielsweise Cercospora canescens;
Pseudocercosporella-Arten, wie beispielsweise Pseudocerco sporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

In Schädlingsbekämpfungsmitteln können die erfindungsgemäßen Wirkstoffe mit besonders gutem Erfolg zur Bekämpfung von Phytophthora-Arten, wie beispielsweise Phytophthora infestans, an Tomaten eingesetzt werden; sowie auch zur Bekämpfung von Plasmopara-Arten, wie beispielsweise Plasmopara viticola, an Reben.

Weiterhin zeigen einige der erfindungsgemäßen Wirkstoffe gute fungizide Wirkung gegen Pyricularia an Reis.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüll massen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle seine.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 Gew.-%, am Wirkungsort erforderlich.

Herstellungsbeispiele

Beispiel 1

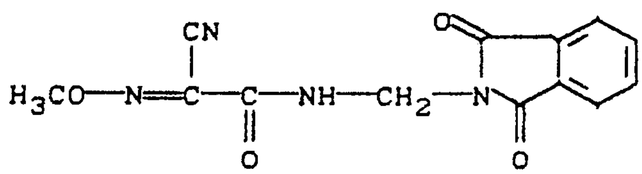

(Verfahren (a))

Zu einer Suspension von 24.0 g (80 mMol) N-Aminomethylphthalimid-hydrochlorid in 80 ml trockenem Dimethylformamid tropft man bei einer Temperatur von 0° C 12,0 g (80 mMol) (E)-2-Cyano-2-methoximinoacetylchlorid und anschließend eine Lösung von 16.0 g (158 mMol) Triethylamin und 1.0 g (8 mMol) 4-Dimethylaminopyridin in 20 ml trockenem Dimethylformamid. Man rührt 1 Stunde bei 0° C und 15 Stunden bei Raumtemperatur, saugt den gebildeten Niederschlag ab, wäscht mit Dimethylformamid und entfernt das Lösungsmittel unter vermindertem Druck. Der Rückstand wird mit 200 ml Essigester und 70 ml Wasser aufgenommen, die organische Phase wird abgetrennt, mit je 70 ml 1 N Salzsäure, gesättigter Natriumhydrogencarbonat-Lösung und Wasser gewaschen und über Natriumsulfat getrockent.

Nach Chromatographie an Kieselgel 60 mit Methylenchlorid als Laufmittel erhält man 8.2 g (30% der Theorie) (E)-2- Cyano-2-methoximino-N-(phthalimidomethyl)-acetamid vom Schmelzpunkt 150-152° C.

Beispiel 2

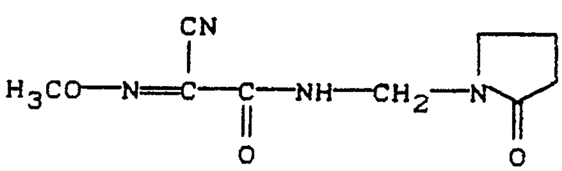

(Verfahren (b))

Man suspendiert 6.55 g (0.05 Mol) (E)-2-Cyano-2-methoximinoacetamid und 5.75 g (0.05 Mol) N-Hydroxymethyl-2-pyrrolidon in 75 ml konzentrierter Essigsäure und tropft bei einer Temperatur von 20-25° C (unter Kühlung) 11.25g (0.11 Mol) konzentrierte Schwefelsäure zu. Anschließend rührt man noch 28 Stunden bei Raumtemperatur.

Das Reaktionsgemisch wird vorsichtig mit 100 ml Eiswasser versetzt und 5mal mit je 100 ml Methylenchlorid extrahiert. Die vereinigten Extrakte werden über Natriumsulfat getrocknet. Nach destillativer

Entfernung des Lösungsmittels wird der Rückstand aus Wasser umkristallisiert.

Man erhält 7.95 g (70% der Theorie) N-[(E)-2-Cyano-2-methoximinoacetamidomethyl)-2-pyrrolidon vom Schmelzpunkt 160-162 ° C.

Beispiel 3

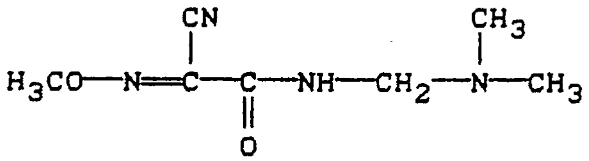

(Verfahren (d))

Eine Mischung aus 38.1 g (0.3 Mol) (E)-2-Cyano-2-methoximinoacetamid, 300 ml tert-Butanol, 79.5 g (0.6 Mol) Dimethylamin (in Form einer 34%igen wäßrigen Lösung) und 48.6 g (046 Mol) Formaldehyd (in Form einer 37%igen wäßrigen Lösung) wird 30 Minuten bei Raumtemperatur und 15 Minuten am Rückfluß erhitzt. Die Lösungsmittel werden destillativ entfernt und der Rückstand aus Petrolether/Ether (1:7) umkristallisiert.

Man erhält 53.8 g (97% der Theorie) (E)-2-Cyano-N-dimethylaminomethyl-2-methoximinoacetamid vom Schmelzpunkt 50-52 ° C.

Analog zu den Beispielen und gemäß den allgemeinen Angaben zu den Verfahren (a), (b), (c), (d) und (e) können die in der nachfolgenden Tabelle 2 angegebenen Verbindungen der Formel (I)

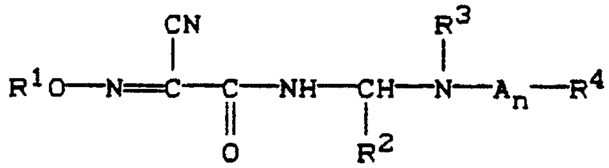

(I)

hergestellt werden.

Tabelle 2

| Bsp.-Nr. | $R^1$ | $R^2$ | 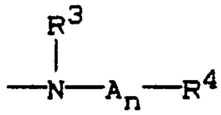 | Schmelzpunkt |
|---|---|---|---|---|
| 4 | $CH_3$ | H | 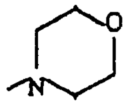 | 134 - 135° C |
| 5 | $CH_3$ | H | 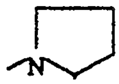 | 68 - 70° C |
| 6 | $CH_3$ | H | 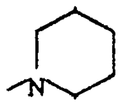 | 116 - 117° C |
| 7 | $CH_3$ | H | 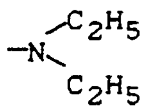 | Öl |
| 8 | $CH_3$ | H | 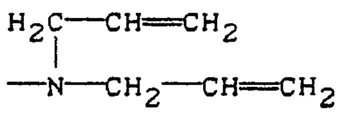 | 50 - 51° C |
| 9 | $CH_3$ | H | 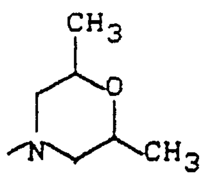 | 102 - 103° C |
| 10 | $CH_3$ | H | 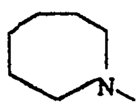 | 99 - 100° C |
| 11 | $CH_3$ | H | 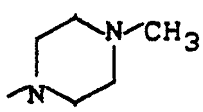 | 99 - 100° C |
| 12 | $CH_3$ | H | 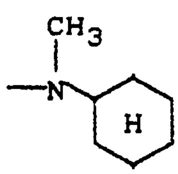 | Öl |

Tabelle 2 - Fortsetzung

| Bsp.-Nr. | $R^1$ | $R^2$ | 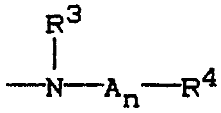 | Schmelzpunkt |
|---|---|---|---|---|
| 13 | $CH_3$ | H | 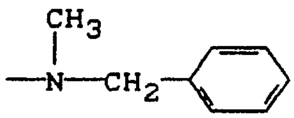 | 62 - 63° C |
| 14 | $CH_3$ | H | 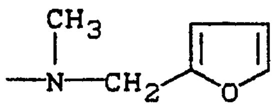 | Öl |
| 15 | $CH_3$ | H | 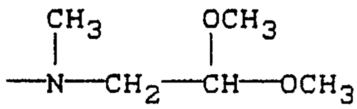 | Öl |
| 16 | $CH_3$ | H | 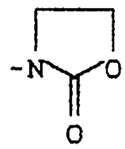 | 125 - 126° C |
| 17 | $CH_3$ | H | 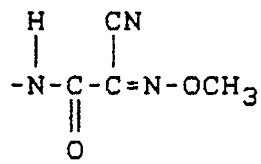 | 142-144° C |
| 18 | $CH_3$ | H | 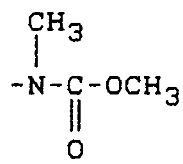 | 131-132° C |
| 19 | $CH_3$ | H | 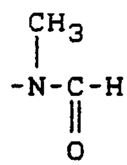 | 117-119° C |
| 20 | $CH_3$ | H | 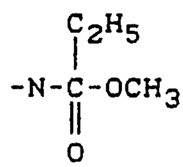 | 63-64° C |

Anwendungsbeispiele

In den folgenden Anwendungsbeispielen wurde die nachstehend aufgeführte Verbindung als Vergleichssubstanzen eingesetzt:

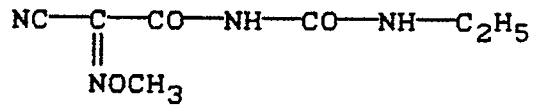

(A)

2-Cyano-N-[(ethylamino)carbonyl]-2-(methoximino)acetamid (bekannt aus DE-OS 23 12 956)

Beispiel A

Phytophthora-Test (Tomate)/kurativ
Lösungsmittel: 4,7 Gewichtsteile Aceton
Emulgator: 0,3 Gewichtsteile Alkyl-aryl-polyglykolether

Zur Herstellung einer zweckmäßigen Wirkstoffzübereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf kurative Wirksamkeit werden junge Pflanzen mit einer wäßrigen Sporensuspension von Phytophthora infestans inokuliert. Die Pflanzen verbleiben 7 Stunden bei 20 °C und 100% relativer Luftfeuchtigkeit in einer Inkubationskabine. Nach einer kurzen Abtrocknungszeit werden die Pflanzen mit der Wirkstoffzübereitung tropfnaß gespritzt.

Die Pflanzen werden in einer Inkubationskabine mit 100% relativer Luftfeuchtigkeit und ca. 20 °C aufgestellt.

3 Tage nach der Inokulation erfolgt die Auswertung.

Eine gute fungizide Wirksamkeit zeigen in diesem Test z.B. die Verbindungen gemäß folgender Herstellungsbeispiele: 10, 11, 12 und 13.

Beispiel B

Plasmopara-Test (Reben)/protektiv
Lösungsmittel: 4,7 Gewichtsteile Aceton
Emulgator: 0,3 Gewichtsteile Alkyl-aryl-polyglykolether

Zur Herstellung einer zweckmäßigen Wirkstoffzübereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzübereitung bis zur Tropfnässe. Nach Antrocknen des Spritzbelages werden die Pflanzen mit einer wäßrigen Sporensuspension von Plasmopara viticola inokuliert und verbleiben dann 1 Tag in einer Feuchtkammer bei 20 bis 22 °C und 100% Luftfeuchtigkeit. Anschließend werden die Pflanzen 5 Tage im Gewächshaus bei 22 °C und ca. 80% Luftfeuchtigkeit aufgestellt. Die Pflanzen werden dann angefeuchtet und 1 Tag in eine Feuchtkammer gestellt.

7 Tage nach der Inokulation erfolgt die Auswertung.

Eine deutliche Überlegenheit in der Wirksamkeit gegenüber dem Stand der Technik zeigen in diesem Test z.B. die Verbindungen gemäß der Herstellungsbeispiele (I) und (2).

## Ansprüche

1. Aminale der allgemeinen Formel (I)

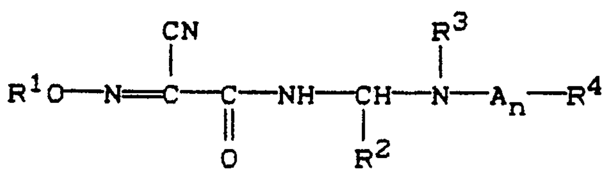

(I)

in welcher
A für -CO-, -CS- oder $-SO_2-$ steht,

n für 0 oder 1 steht,
$R^1$ für gegebenenfalls substituiertes Alkyl steht, wobei als Substituenten genannt seien:
Halogen, Cyano, $-COOR^I$, $-CONR^{II}R^{III}$, $-OR^{IV}$, Acyl, gegebenenfalls substituiertes Aryl, jeweils gegebenenfalls substituiertes Cycloalkyl oder Heterocyclyl;
$R^1$ ferner für jeweils gegebenenfalls substituiertes Alkenyl, Alkinyl, Cycloalkyl oder Cycloalkenyl steht,
$R^2$ für Wasserstoff oder für gegebenenfalls substituiertes Alkyl steht, wobei als Substituenten genannt seien: Cyano, Alkoxy, Alkylthio, $-COOR^I$, Acylamino sowie jeweils gegebenenfalls substituiertes Aryl, Cycloalkyl oder Heterocyclyl;
$R^2$ ferner für jeweils gegebenenfalls substituiertes Alkenyl, Alkinyl, Cycloalkyl oder Cycloalkenyl sowie für jeweils gegebenenfalls substituiertes Aryl oder Heterocyclyl steht, $R^3$ für gegebenenfalls substituiertes Alkyl steht, wobei als Substituenten genannt seien; Halogen, Cyano, $-COOR^I$, $-CONR^{II}R^{III}$, $-OR^{IV}$, die Oxo- oder Thioxogruppe sowie jeweils gegebenenfalls substituiertes Aryl, Cycloalkyl oder Heterocyclyl; ferner für jeweils gegebenenfalls substituiertes Alkenyl, Alkinyl, Cycloalkyl oder Aryl oder für gegebenenfalls substituiertes und gegebenenfalls anelliertes Heterocyclyl sowie für $-OR^V$ steht; weiterhin
$R^3$ für Wasserstoff steht,
für den Fall das entweder n = 1 ist und A für $-SO_2$-steht oder n = 1 ist und $R^4$ für Cycloalkoximino oder Arylthio steht,
$R^4$ für Wasserstoff oder für gegebenenfalls substituiertes Alkyl steht, wobei als Substituenten genannt seien: Halogen, Alkoxy, Alkylthio, Alkylsulfinyl, Alkylsulfonyl, Acyloxy, Acylamino, Cyano, $-COOR^I$, jeweils gegebenenfalls substituiertes Aryl, Cycloalkyl oder Heterocyclyl; ferner für jeweils gegebenenfalls substituiertes Alkenyl oder Alkinyl steht; $R^4$ weiterhin für jeweils gegebenenfalls überbrücktes und/oder anelliertes, jeweils gegebenenfalls substituiertes Cycloalkyl oder Cycloalkenyl steht sowie für jeweils gegebenenfalls anelliertes, jeweils gegebenenfalls substiuiertes Aryl oder Heterocyclyl, für jeweils gegebenenfalls substituiertes Aryloxy oder Arylthio sowie für Alkoxycarbonyl, für 1-Cyanoalkoximino, $-OR^5$, $-SR^5$ und $-NR^6R^7$ steht, oder
$R^3$ und $R^4$ gemeinsam mit dem Stickstoffatom und gegebenenfalls dem Rest A einen gegebenenfalls substituierten 4- bis 9-gliedrigen, gesättigten oder ungesättigten heterocyclischen Ring bilden, welcher gegebenenfalls weitere Heteroatome aus der Reihe Sauerstoff, Schwefel, Stickstoff enthält und welcher gegebenenfalls durch 1 oder 2 gesättigte oder ungesättigte Carbocyclen anelliert ist,
$R^5$ für Wasserstoff, für gegebenenfalls substituiertes Alkyl steht, wobei als Substituenten genannt seien: Halogen, $-OR^{IV}$, $-SR^{IV}$, $-COOR^I$, $-CONR^{II}R^{III}$, -CN, $-NR^{II}R^{III}$, Acyl, jeweils gegebenenfalls substituiertes Aryl oder Aryloxy, jeweils gegebenenfalls substituiertes Cycloalkyl, Cycloalkenyl oder Heterocyclyl; ferner für jeweils gegebenenfalls substituiertes Alkenyl oder Alkinyl sowie für jeweils gegebenenfalls substituiertes Cycloalkyl oder Cycloalkenyl steht,
$R^6$ für Wasserstoff oder für gegebenenfalls substituiertes Alkyl steht, wobei als Substituenten genannt seien: Halogen, Cyano, $-COOR^I$, $-CONR^{II}R^{III}$, $-NR^{II}R^{III}$, $-OR^{IV}$, $-S(O)_nR^{IV}$, Acyl, jeweils gegebenenfalls substituiertes Aryl, Heterocyclyl, Cycloalkyl oder Cycloalkenyl; ferner für jeweils gegebenenfalls substituiertes Alkenyl, Alkinyl, Cycloalkyl, Cycloalkenyl, Aryl oder Heterocyclyl steht und
$R^7$ für die Bedeutung von $R^6$ oder für $-OR^{IV}$ steht, oder
$R^6$ und $R^7$ gemeinsam mit dem Stickstoffatom, an das sie gebunden sind, für einen gegebenenfalls substituierten Heterocyclus stehen, der weitere Heteroatome enthalten kann,
$R^I$ für Wasserstoff oder gegebenenfalls substituiertes Alkyl steht mit folgenden Substituenten: Halogen, jeweils gegebenenfalls substituiertes Aryl, Cycloalkyl und Cycloalkenyl,
$R^{II}$ und $R^{III}$ gleich oder verschieden sind und für Wasserstoff oder gegebenenfalls substituiertes Alkyl stehen mit folgenden Substituenten: Halogen, $-COOR^{IV}$, $-CONR^IR^{IV}$, jeweils gegebenenfalls substituiertes Aryl, Aralkyl, Cycloalkyl und Cycloalkenyl; $R^{II}$ und $R^{III}$ ferner für Alkenyl, Alkinyl oder jeweils gegebenenfalls substituiertes Cycloalkyl, Cycloalkenyl, Aryl oder Heterocyclyl stehen oder gemeinsam mit dem Stickstoffatom, an das sie gebunden sind, für einen gegebenenfalls substituierten Heterocyclus stehen, der weitere Heteroatome enthalten kann,
$R^{IV}$ für Wasserstoff, Alkyl, Aralkyl oder Acyl steht und
$R^V$ für Wasserstoff, Alkyl oder Aralkyl steht.
2. Aminale der Formel (I) gemäß Anspruch 1,
in welcher
A für -CO-, -CS- oder $-SO_2$-,
n für 0 oder 1,
$R^1$ für gegebenenfalls ein- bis fünffach, gleich oder verschieden substituiertes, geradkettiges oder verzweigtes Alkyl mit 1 bis 6 Kohlenstoffatomen, wobei folgende Substituenten infrage kommen: Fluor, Chlor, Brom, Iod, Cyano, $-COOR^I$, $-CONR^{II}R^{III}$,
$-OR^{IV}$, Acyl mit 2 bis 9 Kohlenstoffatomen, gegebenenfalls ein- bis fünffach, gleich oder verschieden

substituiertes Phenyl, wobei als Phenylsubstituenten genannt seien: Halogen, Alkyl und Alkoxy mit jeweils 1 bis 4 Kohlenstoffatomen, Halogenalkyl und Halogenalkoxy mit jeweils 1 oder 2 Kohlenstoffatomen und 1 bis 5 gleichen oder verschiedenen Halogenatomen; weitere Alkylsubstituenten sind gegebenenfalls ein-bis fünffach, gleich oder verschieden durch Alkyl mit 1 bis 4 Kohlenstoffatomen substituiertes Cycloalkyl mit 3 bis 6 Kohlenstoffatomen, gegebenenfalls ein- bis fünffach, gleich oder verschieden durch Halogen und Alkyl mit 1 bis 4 Kohlenstoffatomen substituiertes und gegebenenfalls durch 1 oder 2 6-gliedrige gesättigte oder ungesättigte Carbocyclen anelliertes 3- bis 6-gliedriges Heterocyclyl mit 1 bis 3 gleichen oder verschiedenen Heteroatomen, wie Stickstoff-, Sauerstoff-und Schwefelatomen; $R^1$ steht für jeweils gegebenenfalls ein- bis dreifach, gleich oder verschieden durch Alkyl mit 1 bis 4 Kohlenstoffatomen substituiertes, geradkettiges oder verzweigtes Alkenyl oder Alkinyl mit 2 bis 6 Kohlenstoffatomen; $R^1$ steht für jeweils gegebenenfalls ein- bis fünffach, gleich oder verschieden durch Alkyl mit 1 bis 4 Kohlenstoffatomen substituiertes Cycloalkyl mit 3 bis 6 Kohlenstoffatomen oder Cycloalkenyl mit 5 bis 7 Kohlenstoffatomen;

$R^2$ für Wasserstoff oder für gegebenenfalls einbis fünffach, gleich oder verschieden substituiertes, geradkettiges oder verzweigtes Alkyl mit 1 bis 6 Kohlenstoffatomen, wobei folgende Substituenten infrage kommen: Cyano, Alkoxy und Alkylthio mit jeweils 1 bis 4 Kohlenstoffatomen, $-COOR^{I}$, Acylamino mit 2 bis 9 Kohlenstoffatomen, gegebenenfalls ein- bis fünffach, gleich oder verschieden substituiertes Phenyl, wobei als Substituenten die bei $R^1$ bereits genannten Phenylsubstituenten infrage kommen; weitere Alkylsubstituenten sind gegebenenfalls ein-bis fünffach, gleich oder verschieden durch Alkyl mit 1 bis 4 Kohlenstoffatomen substituiertes Cycloalkyl mit 3 bis 6 Kohlenstoffatomen, gegebenenfalls einbis fünffach, gleich oder verschieden durch Halogen und Alkyl mit 1 bis 4 Kohlenstoffatomen substituiertes und gegebenenfalls durch 1 oder 2 6-gliedrige, gesättigte oder ungesättigte Carbocyclen anelliertes 3- bis 6-gliedriges Heterocyclyl mit 1 bis 3 gleichen oder verschiedenen Heteroatomen, wie Stickstoff-, Sauerstoff- und Schwefelatomen; $R^2$ steht für jeweils gegebenenfalls ein- bis dreifach, gleich oder verschieden substituiertes, geradkettiges oder verzweigtes Alkenyl oder Alkinyl mit 2 bis 6 Kohlenstoffatomen, wobei als Substituenten genannt seien: gegebenenfalls ein- bis fünffach, gleich oder verschieden durch Halogen und Alkyl mit 1 bis 4 Kohlenstoffatomen substituiertes Phenyl; $R^2$ steht für jeweils gegebenenfalls ein- bis fünffach, gleich oder verschieden durch Alkyl mit 1 bis 4 Kohlenstoffatomen substituiertes Cycloalkyl mit 3 bis 6 Kohlenstoffatomen oder Cycloalkenyl mit 5 bis 7 Kohlenstoffatomen; $R^2$ steht für gegebenenfalls ein- bis fünffach, gleich oder verschieden substituiertes Phenyl, wobei als Substituenten die bei $R^2$ bereits genannten Phenylsubstituenten infrage kommen; $R^2$ steht für gegebenenfalls ein-bis fünffach, gleich oder verschieden durch Halogen und Alkyl mit 1 bis 4 Kohlenstoffatomen substituiertes und gegebenenfalls durch 1 oder 2 6-gliedrige, gesättigte oder ungesättigte Carbocyclen anelliertes 3- bis 6-gliedriges Heterocyclyl mit 1 bis 3 gleichen oder verschiedenen Heteroatomen, wie Stickstoff-, Sauerstoff- und Schwefelatome;

$R^3$ für gegebenenfalls ein- bis fünffach, gleich oder verschieden substituiertes, geradkettiges oder verzweigtes Alkyl mit 1 bis 6 Kohlenstoffatomen, wobei folgende Substituenten infrage kommen: Fluor, Chlor, Brom, Iod, Cyano, $-COOR^{I}$, $-CONR^{II}R^{III}$, $-OR^{IV}$, die Oxo-oder Thioxogruppe, gegebenenfalls ein- bis fünffach, gleich oder verschieden substituiertes Phenyl, wobei als Substituenten die bei $R^1$ bereits genannten Phenylsubstituenten infrage kommen; weitere Alkylsubstituenten sind gegebenenfalls ein-bis fünffach, gleich oder verschieden durch Alkyl mit 1 bis 4 Kohlenstoffatomen substituiertes Cycloalkyl mit 3 bis 6 Kohlenstoffatomen sowie gegebenenfalls ein-bis fünffach, gleich oder verschieden durch Halogen, durch Alkyl, Alkoxy oder Alkylthio mit jeweils 1 bis 4 Kohlenstoffatomen, durch Hydroxy, die Oxo- oder Thioxogruppe, Carboxy, Alkoxycarbonyl mit 1 bis 4 Kohlenstoffatomen im Alkoxyteil oder durch Carbamoyl substituiertes 3- bis 6-gliedriges Heterocyclyl mit 1 bis 3 gleichen oder verschiedenen Heteroatomen, wie Stickstoff-, Sauerstoff- und Schwefelatomen; $R^3$ steht für jeweils gegebenenfalls ein- bis dreifach, gleich oder verschieden durch Halogen oder Alkyl mit 1 bis 4 Kohlenstoffatomen substituiertes, geradkettiges oder verzweigtes Alkenyl oder Alkinyl mit jeweils 2 bis 6 Kohlenstoffatomen; $R^3$ steht für jeweils gegebenenfalls ein- bis fünffach, gleich oder verschieden durch Alkyl mit 1 bis 4 Kohlenstoffatomen substituiertes Cycloalkyl mit 3 bis 6 Kohlenstoffatomen; für gegebenenfalls ein- bis fünffach, gleich oder verschieden substituiertes Phenyl, wobei als Substituenten die bei $R^1$ bereits genannten Phenylsubstituenten infrage kommen; $R^3$ steht für gegebenenfalls ein-bis fünffach, gleich oder verschieden durch Halogen, durch Alkyl, Alkoxy oder Alkylthio mit jeweils 1 bis 4 Kohlenstoffatomen, durch Hydroxy, Mercapto, die Oxo-oder Thioxogruppe, durch Carboxy oder Alkoxycarbonyl mit 1 bis 4 Kohlenstoffatomen im Alkoxyteil sowie durch Carbamoyl substituiertes 3-bis 6-gliedriges Heterocyclyl mit 1 bis 3 gleichen oder verschiedenen Heteroatomen, wie Stickstoff-, Sauerstoff- und Schwefelatome, an welches gegebenenfalls 1 oder 2 6-gliedrige gesättigte oder ungesättigte Carbocyclen anelliert sind und weiterhin für $-OR^{V}$;

außerdem steht

$R^3$ für Wasserstoff,

für den Fall das entweder n = 1 ist und A für $-SO_2$-steht oder n = 1 ist und $R^4$ für Cyanalkoximino oder Arylthio steht,

$R^4$ für Wasserstoff oder für gegebenenfalls ein-bis fünffach, gleich oder verschieden substituiertes, geradkettiges oder verzweigtes Alkyl mit 1 bis 6 Kohlenstoffatomen, wobei folgende Substituenten infrage kommen: Fluor, Chlor, Brom, Iod, Cyano, Alkoxy, Alkylthio, Alkylsulfinyl und Alkylsulfonyl mit jeweils 1 bis 4 Kohlenstoffatomen, Acyloxy und Acylamino mit jeweils 2 bis 9 Kohlenstoffatomen, $-COOR^I$, gegebenenfalls ein-bis fünffach, gleich oder verschieden substituiertes Phenyl, wobei als Substituenten die bei $R^1$ bereits genannten Phenylsubstituenten infrage kommen; weitere Alkylsubstituenten sind gegebenenfalls ein-bis fünffach, gleich oder verschieden durch Alkyl mit 1 bis 4 Kohlenstoffatomen substituiertes Cycloalkyl mit 3 bis 6 Kohlenstoffatomen sowie gegebenenfalls ein- bis fünffach, gleich oder verschieden durch Halogen und Alkyl mit 1 bis 4 Kohlenstoffatomen substituiertes und gegebenenfalls durch 1 oder 2 6-gliedrige gesättigte oder ungesättigte Carbocyclen, anelliertes 3-bis 6-gliedriges Heterocyclyl mit 1 bis 3 gleichen oder verschiedenen Heteroatomen, wie Stickstoff-, Sauerstoff- und Schwefelatomen; $R^4$ steht für jeweils gegebenenfalls ein- bis dreifach, gleich oder verschieden substituiertes, geradkettiges oder verzweigtes Alkenyl oder Alkinyl mit jeweils 2 bis 6 Kohlenstoffatomen, wobei als Substi tuenten genannt seien: Alkyl mit 1 bis 4 Kohlenstoffatomen oder gegebenenfalls ein- bis fünffach, gleich oder verschieden durch Halogen und Alkyl mit 1 bis 4 Kohlenstoffatomen substituiertes Phenyl; $R^4$ steht für jeweils gegebenenfalls mit Methylen oder Ethylen überbrücktes und/oder mit 1 oder 2 Benzol-, Cyclopentan- oder Cyclohexanringen anelliertes, jeweils gegebenenfalls ein- bis fünffach, gleich oder verschieden substituiertes Cycloalkyl mit 3 bis 6 Kohlenstoffatomen oder Cycloalkenyl mit 5 bis 7 Kohlenstoffatomen, wobei als Substituenten genannt seien: Halogen, Alkyl und Alkoxy mit jeweils 1 bis 4 Kohlenstoffatomen, Acyloxy und Acylamino mit jeweils 2 bis 5 Kohlenstoffatomen, die Oxogruppe, Phenyl, Hydroxycarbonyl und Alkoxycarbonyl mit 1 bis 4 Kohlenstoffatomen im Alkoxyteil; $R^4$ steht für gegebenenfalls mit 1 oder 2 Benzol- oder Cyclohexanringen anelliertes, gegebenenfalls ein- bis fünffach, gleich oder verschieden substituiertes Phenyl, wobei als Substituenten genannt seien: Halogen, Hydroxy, Nitro, Alkyl und Alkoxy mit jeweils 1 bis 4 Kohlenstoffatomen, Halogenalkyl und Halogenalkoxy mit jeweils 1 oder 2 Kohlenstoffatomen und 2 bis 5 gleichen oder verschiedenen Halogenatomen, wie Fluor- und Chloratome, Acylamino und Acylalkylamino mit jeweils 2 bis 5 Kohlenstoffatomen im Acylteil und 1 bis 4 Kohlenstoffatomen im Alkylteil, Carboxy und Alkoxycarbonyl mit 1 bis 4 Kohlenstoffatomen im Alkoxyteil; $R^4$ steht ferner für jeweils gegebenenfalls ein-bis fünffach, gleich oder verschieden substituiertes Phenoxy oder Phenylthio, wobei als Substituenten die oben genannten Phenylsubstituenten infrage kommen; $R^4$ steht für gegebenenfalls mit 1 oder 2 Benzol-oder Cyclohexanringen anelliertes, gegebenenfalls ein-bis fünffach, gleich oder verschieden substituiertes 5- oder 6-gliedriges Heterocyclyl mit 1 bis 3 gleichen oder verschiedenen Heteroatomen, wie Stickstoff-, Sauerstoff- und Schwefelatome, wobei als Substituenten genannt seien: Halogen, Alkyl mit 1 bis 4 Kohlenstoffatomen, Acyl mit 2 bis 9 Kohlenstoffatomen, Phenyl, die Oxogruppe und Hydroxy; $R^4$ steht auch für Alkoxycarbonyl mit 1 bis 4 Kohlenstoffatomen im Alkoxyteil, für 1-Cyanoalkoximino mit 1 oder 2 Kohlenstoffatomen im Alkoxyteil, für $-OR^5$, $-SR^5$ oder $-NR^6R^7$;

oder

$R^3$ und $R^4$ gemeinsam mit dem Stickstoffatom und gegebenenfalls dem Rest A einen gegebenenfalls ein-bis fünffach, gleich oder verschieden substituiertes und gegebenenfalls durch 1 oder 2 6-gliedrige, gesättigte oder ungesättigte Carbocyclen anellierten 5- bis 7-gliedrigen, gesättigten oder ungesättigten heterocyclischen Ring bilden, der gegebenenfalls weitere 1 oder 2 gleiche oder verschiedene Heteroatomen aus der Reihe Sauerstoff, Schwefel, Stickstoff enthält, wobei als Substituenten genannt seien: Halogen, Alkyl, Alkylthio oder Alkoxy mit jeweils 1 bis 4 Kohlenstoffatomen, Hydroxy, Mercapto, die Oxo-oder Thioxogruppe, Carboxy, Alkoxycarbonyl mit 1 bis 4 Kohlenstoffatomen im Alkoxyteil, Carbamoyl, (Di)-alkylcarbamoyl mit 1 bis 4 Kohlenstoffatomen in den jeweiligen Alkylteilen, gegebenenfalls ein-bis fünffach, gleich oder verschieden substituiertes Phenyl, wobei als Substituenten Halogen und Alkyl mit 1 bis 4 Kohlenstoffatomen infrage kommen sowie gegebenenfalls ein- bis fünffach, gleich oder verschieden substituiertes 5- oder 6-gliedriges Heterocyclyl mit 1 bis 3 gleichen oder verschiedenen Heteroatomen aus der Reihe Sauerstoff, Schwefel, Stickstoff, wobei als Substituenten Halogen und Alkyl mit 1 bis 4 Kohlenstoffatomen infrage kommen;

$R^5$ für Wasserstoff, für gegebenenfalls ein- bis fünffach, gleich oder verschieden substituiertes, geradkettiges oder verzweigtes Alkyl mit 1 bis 6 Kohlenstoffatomen, wobei folgende Substituenten infrage kommen: Halogen, $-OR^{IV}$, $-SR^{IV}$, $-COOR^I$, $-CONR^{II}R^{III}$, CN, $-NR^{II}R^{III}$, Acyl mit 2 bis 9 Kohlenstoffatomen, jeweils gegebenenfalls einfach bis fünffach, gleich oder verschieden durch Halogen und Alkyl mit 1 bis 4 Kohlenstoffatomen substituiertes Phenyl oder Phenoxy, jeweils gegebenenfalls ein-bis fünffach, gleich oder verschieden durch Alkyl mit 1 bis 4 Kohlenstoffatomen substituiertes Cycloalkyl mit 3 bis 6 Kohlenstoffatomen oder Cycloalkenyl mit 5 bis 7 Kohlenstoffatomen, gegebenenfalls ein- bis fünffach, gleich oder

verschieden durch Halogen und Alkyl mit 1 bis 4 Kohlenstoffatomen substituiertes 3- bis 6-gliedriges Heterocyclyl mit 1 bis 3 gleichen oder verschiedenen Heteroatomen, wie Stickstoff-, Sauerstoff- und Schwefelatomen; $R^5$ steht für jeweils gegebenenfalls ein- bis fünffach, gleich oder verschieden durch Alkyl mit 1 bis 4 Kohlenstoffatomen substituiertes Alkenyl oder Alkinyl mit jeweils 2 bis 6 Kohlenstoffatomen, für jeweils gegebenenfalls ein- bis fünffach, gleich oder verschieden durch Alkyl mit 1 bis 4 Kohlenstoffatomen substituiertes Cycloalkyl mit 3 bis 6 Kohlenstoffatomen oder Cycloalkenyl mit 5 bis 7 Kohlenstoffatomen;

$R^6$ für Wasserstoff oder für gegebenenfalls gleich oder verschieden, ein- oder zweifach substituiertes, geradkettiges oder verzweigtes Alkyl mit 1 bis 4 Kohlenstoffatomen, wobei als Substituenten genannt seien: Halogen, Cyano, $COOR^I$, $-CONR^{II}R^{III}$, $-NR^{II}R^{III}$, $-OR^{IV}$, $-S(O)_nR^{IV}$, Acyl mit 2 bis 9 Kohlenstoffatomen, gegebenenfalls ein- bis fünffach, gleich oder verschieden durch Halogen und Alkyl mit 1 bis 4 Kohlenstoffatomen substituiertes Phenyl, gegebenenfalls ein- bis fünffach, gleich oder verschieden durch Halogen, Alkyl und Alkoxy mit jeweils 1 bis 4 Kohlenstoffatomen substituiertes 5- oder 6-gliedriges Heterocyclyl mit 1 bis 3 gleichen oder verschiedenen Heteroatomen, z. B. Stickstoff, Sauerstoff und Schwefel, jeweils gegebenenfalls ein- bis fünffach, gleich oder verschieden durch Alkyl mit 1 bis 4 Kohlenstoffatomen substituiertes Cycloalkyl mit 3 bis 6 Kohlenstoffatomen oder Cycloalkenyl mit 5 bis 7 Kohlenstoffatomen; $R^6$ steht für geradkettiges oder verzweigtes Alkenyl oder Alkinyl mit jeweils 2 bis 6 Kohlenstoffatomen; für gegebenenfalls ein- bis fünffach, gleich oder verschieden durch Alkyl mit 1 bis 4 Kohlenstoffatomen substituiertes Cycloalkenyl mit 5 bis 7 Kohlenstoffatomen oder für gegebenenfalls ein- bis fünffach, gleich oder verschieden substituiertes Cyloalkyl mit 3 bis 6 Kohlenstoffatomen, wobei als Substituenten genannt seien: Halogen, Alkyl und Alkoxy mit jeweils 1 bis 4 Kohlenstoffatomen, Cyano, Amino, Carbamoyl, Alkylamino, Dialkylamino, Alkylcarbamoyl und Dialkylcarbamoyl mit jeweils 1 bis 4 Kohlenstoffstomen in jedem Alkylteil, Alkoxycarbonylamino mit 1 bis 4 Kohlenstoffatomen im Alkoxyteil, Cycloalkyl und Cycloalkylalkyl mit 5 oder 6 Kohlenstoffatomen im Cycloalkylteil und 1 bis 4 Kohlenstoffatomen im geradkettigen oder verzweigten Alkylteil, gegebenenfalls ein- bis fünffach, gleich oder verschieden durch Halogen und Alkyl mit 1 bis 4 Kohlenstoffatomen substituiertes Phenyl oder Pyrrolidon; $R^6$ steht für gegebenenfalls ein- bis fünffach, gleich oder verschieden durch Halogen, Alkyl oder Alkoxy mit jeweils 1 bis 4 Kohlenstoffatomen, Halogenalkyl oder Halogenalkoxy mit jeweils 1 oder 2 Kohlenstoffatomen und 1 bis 5 gleichen oder verschiedenen Halogenatomen substituiertes Phenyl oder für einen gegebenenfalls ein-bis fünffach, gleich oder verschieden substituierten 5-oder 6-gliedrigen Heterocyclus mit 1 bis 3 gleichen oder verschiedenen Heteroatomen, wie insbesondere Stickstoff-, Sauerstoff- und Schwefelatomen; als Substituenten seien genannt: Halogen, Mercapto, Phenyl, geradkettiges oder verzweigtes Alkyl, Alkoxy, Alkylthio, Alkylsulfinyl und Alkylsulfonyl mit 1 bis 4 Kohlenstoffatomen je Alkylteil;

$R^7$ für die Bedeutungen von $R^6$ oder die Gruppierung $-OR^{IV}$;

oder

$R^6$ und $R^7$ gemeinsam mit dem Stickstoffatom, an das sie gebunden sind, für einen gegebenenfalls ein- bis fünffach, gleich oder verschieden substituierten mono-, bi- oder tricyclischen Heterocyclus oder Spiroheterocyclus mit ein bis drei weiteren, gleichen oder verschiedenen Heteroatomen, wie Sauerstoff-, Stickstoff- oder Schwefelatome, wobei als Substituenten genannt seien:

geradkettiges oder verzweigtes Alkyl oder Alkoxy mit jeweils 1 bis 4 Kohlenstoffatomen, Cycloalkyl mit 3 bis 6 Kohlenstoffatomen, die Hydroxy- oder die Oxo-Gruppe, geradkettiges oder verzweigtes Alkenyl mit 2 bis 4 Kohlenstoffatomen, Carboxy, Alkoxycarbonyl mit 1 bis 4 Kohlenstoffatomen im geradkettigen oder verzweigten Alkylteil, Carbamoyl, Alkyl-oder Dialkylcarbamoyl mit jeweils 1 bis 4 Kohlenstoffatomen in jedem geradkettigen oder verzweigten Alkylteil, jeweils gegebenenfalls ein- oder zweifach, gleich oder verschieden durch Halogen und geradkettiges oder verzweigtes Alkyl und Alkoxy mit jeweils 1 bis 4 Kohlenstoffatomen substituiertes Phenyl oder Phenylalkyl mit 1 bis 4 Kohlenstoffatomen im geradkettigen oder verzweigten Alkylteil;

$R_I$ für Wasserstoff oder geradkettiges oder verzweigtes Alkyl mit 1 bis 6 Kohlenstoffatomen,

$R^{II}$ und $R^{III}$ gleich oder verschieden sind und für Wasserstoff, geradkettiges oder verzweigtes Alkyl mit 1 bis 6 Kohlenstoffatomen, gegebenenfalls ein- bis fünffach, gleich oder verschieden substituiertes Phenylalkyl mit 1 bis 4 Kohlenstoffatomen im geradkettigen oder verzweigten Alkylteil, wobei als Phenylsubstituenten genannt seien: Halogen, Alkyl oder Alkoxy mit jeweils 1 bis 4 Kohlenstoffatomen, Halogenalkyl oder Halogenalkoxy mit jeweils 1 oder 2 Kohlenstoffatomen und 2 bis 5 gleichen oder verschiedenen Halogenatomen; $R^{II}$ steht ferner für Alkoxycarbonylalkyl, Carbamoylalkyl, Alkylcarbamoylalkyl oder Dialkylcarbamoylalkyl mit jeweils 1 bis 4 Kohlenstoffatomen in jedem Alkylteil oder für gegebenenfalls ein- bis fünffach, gleich oder verschieden durch geradkettiges oder verzweigtes Alkyl mit 1 bis 4 Kohlenstoffatomen substituiertes Cycloalkyl mit 3 bis 6 Kohlenstoffatomen stehen;

$R^{IV}$ für Wasserstoff, geradkettiges oder verzweigtes Alkyl mit 1 bis 6 Kohlenstoffatomen, gegebenenfalls ein- bis fünffach, gleich oder verschieden substituiertes Phenylalkyl mit 1 bis 4 Kohlenstoffatomen im geradkettigen oder verzweigten Alkylteil, wobei als Phenylsubstituenten die bei $R^{II}$ genannten Phenylsubstituenten infrage kommen; $R^{II}$ steht ferner für Acyl mit 2 bis 9 Kohlenstoffatomen;

$R^{V}$ für Wasserstoff, geradkettiges oder verzweigtes Alkyl mit 1 bis 6 Kohlenstoffatomen oder für gegebenenfalls ein- bis fünffach, gleich oder verschieden substituiertes Phenylalkyl mit 1 bis 4 Kohlenstoffatomen im geradkettigen oder verzweigten Alkylteil, wobei als Phenylsubstituenten die bei $R^{II}$ genannten Phenylsubstituenten infrage kommen.

3. Aminale der allgemeinen Formel (I) gemäß Anspruch 1,
in welcher
A für -CO-, -CS- oder $-SO_2-$ steht,
n für 0 oder 1 steht,
$R^1$ für gegebenenfalls ein- bis dreifach, gleich oder verschieden substituiertes, geradkettiges oder verzweigtes Alkyl mit 1 bis 4 Kohlenstoffstomen steht, wobei folgende Substituenten infrage kommen: Fluor, Chlor, Brom, Iod, Cyano, $-COOR^{I}$, $-CONR^{II}R^{III}$, $-OR^{IV}$ Acyl mit 2 bis 9 Kohlenstoffatomen, gegebenenfalls einbis dreifach, gleich oder verschieden durch Fluor, Chlor, Methyl und Methoxy substituiertes Phenyl, jeweils gegebenenfalls ein- bis dreifach durch Methyl substituiertes Cyclopropyl oder Cyclohexyl, gegebenenfalls ein- bis dreifach, gleich oder verschieden durch Alkyl mit 1 oder 2 Kohlenstoffstomen substituierte Heterocyclen der Formeln

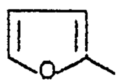 , 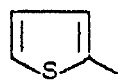 , 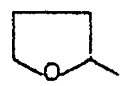 , 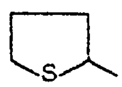 , 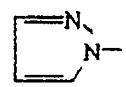

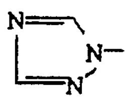 , 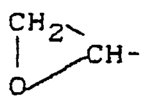 , 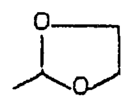 und 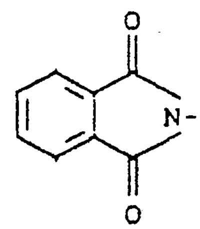

$R^1$ steht ferner für gegebenenfalls ein- oder zweifach durch Methyl substituiertes Allyl oder Propargyl, für jeweils gegebenenfalls ein- bis dreifach durch Methyl substituiertes Cyclopropyl, Cyclohexyl, Cyclopentenyl oder Cyclohexenyl;

$R^2$ für Wasserstoff oder für gegebenenfalls einbis dreifach, gleich oder verschieden substituiertes, geradkettiges oder verzweigtes Alkyl mit 1 bis 4 Kohlenstoffatomen steht, wobei folgende Substituenten infrage kommen: Cyano, Methoxy, Ethoxy, Methylthio, Ethylthio, $-COOR^{I}$, Acylamino mit 2 bis 9 Kohlenstoffatomen, gegebenenfalls ein- bis dreifach, gleich oder verschieden durch Halogen, Methyl und Methoxy substituiertes Phenyl, jeweils gegebenenfalls ein- bis dreifach durch Methyl substituiertes Cyclopropyl oder Cyclohexyl sowie gegebenenfalls ein- bis dreifach, gleich oder verschieden durch Alkyl mit 1 oder 2 Kohlenstoffatomen substituierte Heterocyclen der Formeln

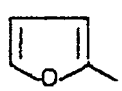 , 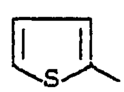 , 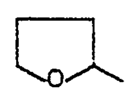 , 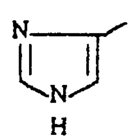 ,

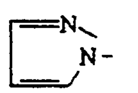 , 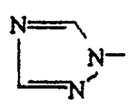 , 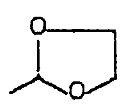 und 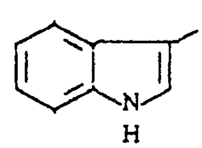 ;

$R^2$ steht ferner für jeweils gegebenenfalls durch Phenyl, das gegebenenfalls ein- bis dreifach, gleich oder verschieden durch Halogen und Methyl substituiert sein kann, substituiertes Allyl, Allenyl, Vinyl, Propargyl oder Ethinyl: $R^2$ steht außerdem für Cyclopropyl, Cyclohexyl, Cyclopentenyl oder Cyclohexenyl; $R^2$ steht weiterhin für gegebenenfalls ein- bis dreifach, gleich oder verschieden durch Halogen, Methyl und Methoxy

substituiertes Phenyl, für gegebenenfalls ein- bis dreifach, gleich oder verschieden durch Alkyl mit 1 oder 2 Kohlenstoffatomen substituierte Heterocyclen der Formeln

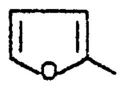 , 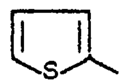 , 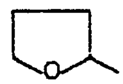 , 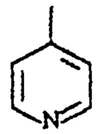 , 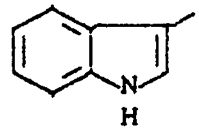 ,

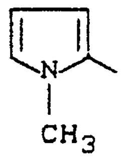 , 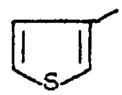 ;

$R^3$ für gegebenenfalls ein- bis dreifach, gleich oder verschieden substituiertes, geradkettiges oder verzweigtes Alkyl mit 1 bis 4 Kohlenstoffatomen steht, wobei folgende Substituenten infrage kommen: Fluor, Chlor, Brom, Iod, Cyano, $-COOR^{I}$, $-CONR^{II}R^{III}$, $-OR^{IV}$, die Oxo- oder Thioxogruppe, gegebenenfalls ein- bis dreifach, gleich oder verschieden, durch Fluor, Chlor, Methyl und Methoxy substituiertes Phenyl; weitere Alkylsubstituenten sind jeweils gegebenenfalls ein- bis dreifach, gleich oder verschieden durch Halogen oder Methyl substituiertes Cyclopropyl, Cyclopentyl oder Cyclohexyl, gegebenenfalls ein- bis dreifach, gleich oder verschieden, durch Chlor, Methyl, Methoxy, Hydroxy, Methylthio, die Oxo- oder Thioxogruppe, Carboxy, Methoxycarbonyl, Ethoxycarbonyl sowie Carbamoyl substituiertes Heterocyclyl der Formeln:

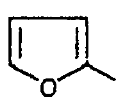 , 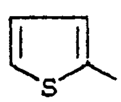 , 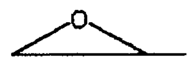 , 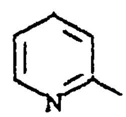 , 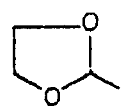

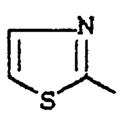 , 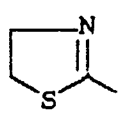 , 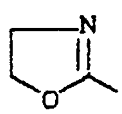 , 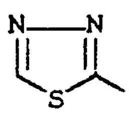 , 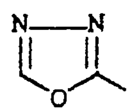

$R^3$ steht ferner für gegebenenfalls ein- bis zweifach durch Halogen oder Methyl substituiertes Allyl oder Propargyl; für jeweils gegebenenfalls ein- bis dreifach durch Methyl substituiertes Cyclopropyl, Cyclopentyl oder Cyclohexyl; für gegebenenfalls ein- bis dreifach gleich oder verschieden durch Fluor, Chlor, Methyl oder Methoxy substituiertes Phenyl; sowie für gegebenenfalls ein- bis dreifach, gleich oder verschieden, durch Fluor, Chlor, Methyl, Methoxy, Methylthio, Hydroxy, Mercapto, die Oxo- oder Thioxogruppe, durch Carboxy, Methoxycarbonyl, Ethoxycarbonyl sowie durch Carbamoyl substituierte Heterocyclen der Formeln

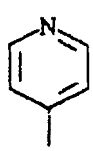 , 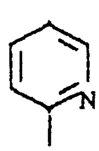 , 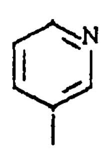 , 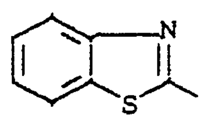 , 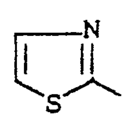

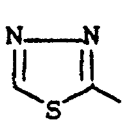 , 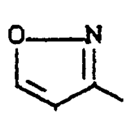 , 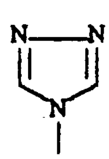 , 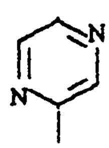 , 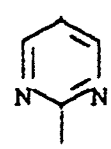

und weiterhin für $OR^{V}$;
außerdem steht

$R^3$ für Wasserstoff

für den Fall daß entweder n = 1 ist und A für $-SO_2$-steht oder n = 1 ist und $R^4$ für Cyanmethoximino oder Phenylthio steht,

$R^4$ für Wasserstoff oder für gegebenenfalls ein-bis dreifach, gleich oder verschieden substituiertes, geradkettiges oder verzweigtes Alkyl mit 1 bis 4 Kohlenstoffatomen steht, wobei folgende Substituenten infrage kommen: Fluor, Chlor, Brom, Iod, Cyano, Alkoxy, Alkylthio, Alkylsulfinyl und Alkylsulfonyl mit jeweils 1 oder 2 Kohlenstoffatomen, Acyloxy und Acylamino mit jeweils 2 bis 9 Kohlenstoffatomen, -COOR', gegebenenfalls ein-bis dreifach, gleich oder verschieden durch Halogen und Methyl substituiertes Phenyl, Cyclopropyl, Cyclopentyl oder Cyclohexyl, gegebenenfalls ein- bis dreifach, gleich oder verschieden durch Alkyl mit 1 oder 2 Kohlenstoffatomen substituierte Heterocyclen der Formeln

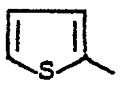, 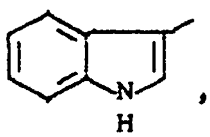, 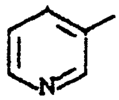, 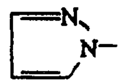,

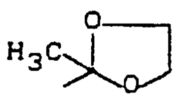, 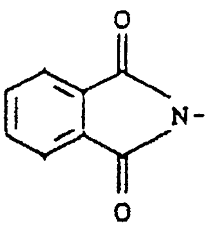, 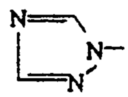, 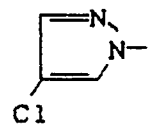,

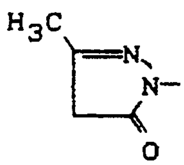, 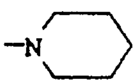, 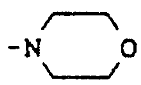;

$R^4$ steht ferner für jeweils gegebenenfalls durch Methyl oder Phenyl, das gegebenenfalls ein- bis dreifach, gleich oder verschieden durch Halogen und Methyl substituiert sein kann, substituiertes Vinyl, Allyl oder Ethinyl; $R^4$ steht weiterhin für jeweils gegebenenfalls mit Methylen oder Ethylen überbrücktes und/oder mit 1 oder 2 Benzol-, Cyclopentan- oder Cyclohexanringen anelliertes, jeweils gegebenenfalls ein- bis dreifach, gleich oder verschieden substituiertes Cyclopropyl, Cyclopentyl, Cyclohexyl, Cyclopentenyl oder Cyclohexenyl, wobei als Substituenten genannt seien: Halogen, Methyl, Methoxy, Acyloxy und Acylamino mit jeweils 2 bis 5 Kohlenstoffatomen, die Oxogruppe, Phenyl, Hydroxycarbonyl, Methoxy- und Ethoxycarbonyl; $R^4$ steht außerdem für gegebenenfalls mit 1 oder 2 Benzol- oder Cyclohexanringen anelliertes, gegebenenfalls ein- bis dreifach, gleich oder verschieden substituiertes Phenyl, wobei als Substituenten genannt seien: Fluor, Chlor, Hydroxy, Nitro, Methyl, Methoxy, Acylamino und N-Alkyl-acyl-amino mit jeweils 2 bis 5 Kohlenstoffatomen im Acylteil und 1 oder 2 Kohlenstoffatomen im Alkylteil, Carboxy, Methoxy- und Ethoxycarbonyl; $R^4$ steht für jeweils gegebenenfalls ein-bis dreifach, gleich oder verschieden substituiertes Phenoxy oder Phenylthio, wobei als Substituenten die oben genannten Phenylsubstituenten infrage kommen; $R^4$ steht ferner für gegebenenfalls mit 1 oder 2 Benzol- oder Cyclohexanringen anelliertes, gegebenenfalls ein- bis dreifach, gleich oder verschieden substituiertes 5- oder 6-gliedriges Heterocyclyl mit 1 bis 3 gleichen oder verschiedenen Heteroatomen, wie Stickstoff-, Sauerstoff- und Schwefelatomen, wobei als Substituenten genannt seien: Acyl mit 2 bis 5 Kohlenstoffatomen, Chlor, Brom, Methyl, Ethyl, Phenyl, Oxo und Hydroxy; $R^4$ steht schließlich auch für Alkoxycarbonyl mit 1 oder 2 Kohlenstoffatomen im Alkoxyteil, für 1-Cyanomethoximino, $-OR^5$, $-SR^5$ oder $-NR^6R^7$;

oder

$R^3$ und $R^4$ gemeinsam für jeweils gegebenenfalls ein-bis fünffach, gleich oder verschieden, durch Methyl, Methoxy, Methylthio, Hydroxy, Mercapto, die Oxo-oder Thioxogruppe, Carboxy, Methoxycarbonyl, Ethoxycarbonyl, Carbamoyl oder Phenyl substituiertes Heterocyclyl der Formeln

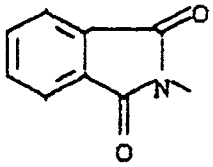 , 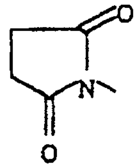 , 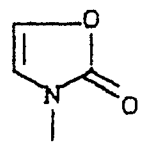 , 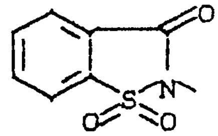 ,

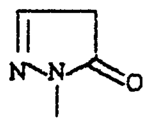 , 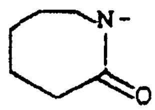 , 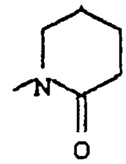 , 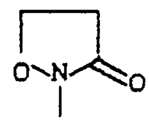 ,

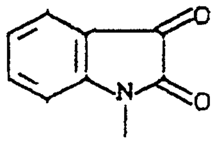 , 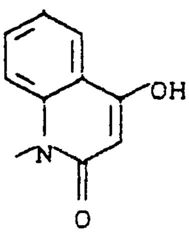 , 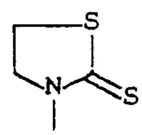 , 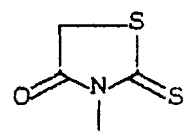 ,

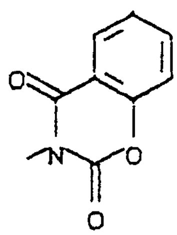 , 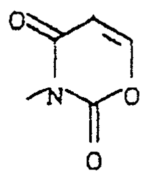 , 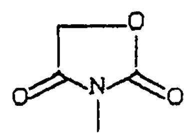 , 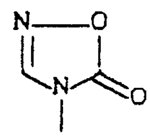 ,

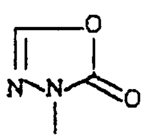 , 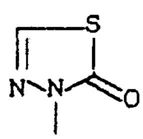 ,

vorzugsweise der Formeln

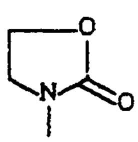 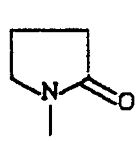 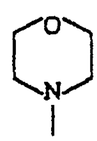

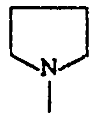
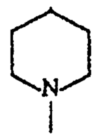
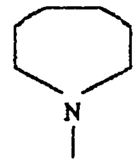

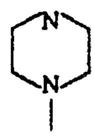

$R^5$ für Wasserstoff, für gegebenenfalls ein- bis dreifach, gleich oder verschieden substituiertes, geradkettiges oder verzweigtes Alkyl mit 1 bis 4 Kohlenstoffatomen steht, wobei folgende Substituenten infrage kommen: Fluor, Chlor, $-OR^{IV}$, $-SR^{IV}$, $-COOR^{I}$, $-CONR^{II}R^{III}$, CN, $NR^{II}R^{III}$ Alkylcarbonyl mit 1 bis 4 Kohlenstoffatomen im geradkettigen oder verzweigten Alkylteil, jeweils gegebenenfalls ein- bis dreifach, gleich oder verschieden durch Halogen und Methyl substituiertes Phenyl oder Phenoxy, jeweils gegebenenfalls ein- bis dreifach durch Methyl substituiertes Cyclopropyl, Cyclohexyl, Cyclopentenyl oder Cyclohexenyl, gegebenenfalls ein-bis dreifach, gleich oder verschieden durch Halogen, Methyl und Ethyl substituiertes 3- bis 6-gliedriges Heterocyclyl mit 1 bis 3 gleichen oder verschiedenen Heteroatomen, wie Stickstoff-, Sauerstoff- und Schwefelatomen;

$R^5$ steht ferner für gegebenenfalls ein- oder zweifach durch Methyl substituiertes Allyl oder Propargyl sowie für jeweils gegebenenfalls einbis dreifach durch Methyl substituiertes Cyclopropyl, Cyclohexyl, Cyclopentenyl oder Cyclohexenyl;

$R^6$ für Wasserstoff oder für gegebenenfalls ein-oder zweifach, gleich oder verschieden substituiertes geradkettiges oder verzweigtes Alkyl mit 1 bis 4 Kohlenstoffatomen steht, wobei als Substituenten vorzugsweise genannt seien: Halogen, Cyano, $-COOR^{I}$, $CONR^{II}R^{III}$, $NR^{II}R^{III}$, $-OR^{IV}$, $-S(O)_nR^{IV}$, Acyl mit 2 bis 9 Kohlenstoffatomen, gegebenenfalls ein- bis dreifach, gleich oder verschieden durch Halogen und Alkyl mit 1 bis 4 Kohlenstoffatomen substituiertes Phenyl, gegebenenfalls ein- bis dreifach, gleich oder verschieden durch Halogen, geradkettiges oder verzweigtes Alkyl und Alkoxy mit jeweils , bis 4 Kohlenstoffatomen substituiertes 5- oder 6-gliedriges Heterocyclyl mit 1 bis 3 gleichen oder verschiedenen Heteroatomen, insbesondere Stickstoff, Sauerstoff und Schwefel, und jeweils gegebenenfalls ein- bis dreifach, gleich oder verschieden durch geradkettiges oder verzweigtes Alkyl mit 1 bis 4 Kohlenstoffatomen substituiertes Cycloalkyl mit 3 bis 6 Kohlenstoffatomen oder Cycloalkenyl mit 5 bis 7 Kohlenstoffatomen; $R^6$ steht für geradkettiges oder verzweigtes Alkenyl oder Alkinyl mit jeweils 2 bis 6 Kohlenstoffatomen; für gegebenenfalls ein- bis dreifach, gleich oder verschieden durch geradkettiges oder verzweigtes Alkyl mit 1 bis 4 Kohlenstoffatomen substituiertes Cycloalkenyl mit 5 bis 7 Kohlenstoffatomen oder für gegebenenfalls ein-bis dreifach, gleich oder verschieden substituiertes Cycloalkyl mit 3 bis 6 Kohlenstoffatomen, wobei als Substituenten vorzugsweise genannt seien: Halogen, geradkettiges oder verzweigtes Alkyl und Alkoxy mit jeweils 1 bis 4 Kohlenstoffatomen, Cyano, Amino, Carbamoyl, jeweils geradkettiges oder verzweigtes Alkylamino, Dialkylamino, Alkylcarbamoyl und Dialkylcarbamoyl mit jeweils 1 bis 4 Kohlenstoffatomen in jedem Alkylteil, Alkoxycarbonylamino mit 1 bis 4 Kohlenstoffatomen im geradkettigen oder verzweigten Alkoxyteil, Cycloalkyl und Cycloalkylalkyl mit jeweils 5 oder 6 Kohlenstoffatomen im Cycloalkylteil und 1 bis 4 Kohlenstoffatomen im geradkettigen oder verzweigten Alkylteil, gegebenenfalls ein- bis dreifach, gleich oder verschieden durch Halogen und Alkyl mit 1 bis 4 Kohlenstoffatomen substituiertes Phenyl oder Pyrrolidon; $R^6$ steht für gegebenenfalls einbis dreifach, gleich oder verschieden durch Halogen, geradkettiges oder verzweigtes Alkyl oder Alkoxy mit jeweils 1 bis 4 Kohlenstoff atomen, Halogenalkyl oder Halogenalkoxy mit jeweils 1 oder 2 Kohlenstoffatomen und 1 bis 5 gleichen oder verschiedenen Halogenatomen substituiertes Phenyl oder für einen gegebenenfalls ein- bis dreifach, gleich oder verschieden substituierten 5- oder 6-gliedrigen Heterocyclus mit 1 bis 3 gleichen oder verschiedenen Heteroatomen, wie insbesondere Stickstoff, Sauerstoff oder Schwefel; als Substituenten für die Heterocyclen seien genannt: Halogen, Mercapto, Phenyl, geradkettiges oder verzweigtes Alkyl, Alkoxy, Alkylthio, Alkylsulfinyl und Alkylsulfonyl mit 1 bis 4 Kohlenstoffatomen je Alkylteil;

$R^7$ für die Bedeutungen von $R^6$ oder die Gruppierung $-OR^{IV}$ steht,

oder

$R^6$ und $R^7$gemeinsam mit dem Stickstoffatom, an das sie gebunden sind, für einen gegebenenfalls ein-bis fünffach, gleich oder verschieden substituierten mono-, bi- oder tricyclischen Heterocyclus oder Spiroheterocyclus stehen, wobei als Heterocyclen genannt seien: Oxazolidin, Pyrrolidin, Imidazolidin, Piperidin, Piperazin, Morpholin, Thiomorpholin, 1,3-Oxazan oder 1,3-Diazan; diese Heterocyclen können jeweils gegebenenfalls mit 1 oder 2 Benzoloder Cyclohexanringen anelliert sein oder gegebenenfalls mit Methylen oder Ethylen überbrückt sein;
als Substituenten für alle Heterosysteme seien genannt:
geradkettiges oder verzweigtes Alkyl oder Cycloalkyl mit 3 bis 6 Kohlenstoffatomen, die Hydroxy- oder die Oxo-Gruppe, geradkettiges oder verzweigtes Alkenyl mit 2 bis 4 Kohlenstoffatomen, Carboxy, Alkoxycarbonyl mit 1 bis 4 Kohlenstoffatomen im geradkettigen oder verzweigten Alkylteil, Carbamoyl, Alkyl- oder Dialkylcarbamoyl mit jeweils 1 bis 4 Kohlenstoffatomen in jedem Alkylteil, ferner jeweils gegebenenfals ein- oder zweifach, gleich oder verschieden durch Halogen und geradkettiges oder verzweigtes Alkyl und Alkoxy mit jeweils 1 bis 4 Kohlenstoffatomen substituiertes Phenyl oder Phenylalkyl mit 1 bis 4 Kohlenstoffatomen im geradkettigen oder verzweigten Alkylteil,
$R^I$ für Wasserstoff oder geradkettiges oder verzweigtes Alkyl mit 1 bis 4 Kohlenstoffatomen steht,
$R^{II}$ und $R^{III}$ gleich oder verschieden sind und für Wasserstoff, geradkettiges oder verzweigtes Alkyl mit 1 bis 4 Kohlenstoffatomen, jeweils gegebenenfalls ein- bis dreifach, gleich oder verschieden substituiertes Benzyl oder Phenethyl steht, wobei als Phenylsubstituenten jeweils genannt seien: Fluor, Chlor, Methyl, Methoxy und Trifluormethyl; $R^{II}$ ferner für Alkoxycarbonylalkyl mit 1 bis 4 Kohlenstoff atomen im Alkoxyteil und 1 oder 2 Kohlenstoffatomen im Alkylteil, Carbamoylalkyl mit 1 oder 2 Kohlenstoffatomen im Alkylteil, Alkylcarbamoylalkyl oder Dialkylcarbamoylalkyl mit jeweils 1 oder 2 Kohlenstoffatomen in jedem Alkylteil, oder für gegebenenfalls ein- bis dreifach, gleich oder verschieden durch Methyl und Ethyl substituiertes Cycloalkyl mit 3 bis 6 Kohlenstoffatomen stehen,
$R^{IV}$ für Wasserstoff, geradkettiges oder verzweigtes Alkyl mit 1 bis 4 Kohlenstoffatomen, jeweils gegebenenfalls ein- bis dreifach, gleich oder verschieden substituiertes Benzyl oder Phenethyl steht, wobei als Phenylsubstituenten jeweils genannt seien: Fluor, Chlor, Methyl, Methoxy und Trifluormethyl; $R^{IV}$ steht ferner für Acyl mit 2 bis 9 Kohlenstoffatomen;
$R^V$ für Wasserstoff, geradkettiges oder verzweigtes Alkyl mit 1 bis 4 Kohlenstoffatomen oder für jeweils gegebenenfalls ein- bis dreifach gleich oder verschieden substituiertes Benzyl oder Phenethyl steht, wobei als Phenylsubstituenten die bei $R^{II}$ genannten Phenylsubstituenten infrage kommen.
4. Verfahren zur Herstellung von substituierten Aminalen der allgemeinen Formel (I)

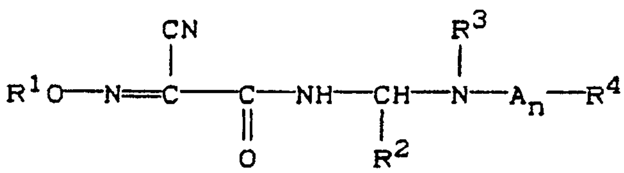

(I)

in welcher
A für -CO-, -CS- oder $-SO_2-$ steht,
n für 0 oder 1 steht,
$R^1$ für gegebenenfalls substituiertes Alkyl steht, wobei als Substituenten genannt seien: Halogen, Cyano, $-COOR^I$, $-CONR^{II}R^{III}$, $-OR^{IV}$ Acyl, gegebenenfalls substituiertes Aryl, jeweils gegebenenfalls substituiertes Cycloalkyl oder Heterocyclyl;
$R^1$ ferner für jeweils gegebenenfalls substituiertes Alkenyl, Alkinyl, Cycloalkyl oder Cycloalkenyl steht,
$R^2$ für Wasserstoff oder für gegebenenfalls substituiertes Alkyl steht, wobei als Substituenten genannt seien: Cyano, Alkoxy, Alkylthio, $COOR^I$, Acylamino sowie jeweils gegebenenfalls substituiertes Aryl, Cycloalkyl oder Heterocyclyl;
$R^2$ ferner für jeweils gegebenenfalls substituiertes Alkenyl, Alkinyl, Cycloalkyl oder Cycloalkenyl sowie für jeweils gegebenenfalls substituiertes Aryl oder Heterocyclyl steht,
$R^3$ für gegebenenfalls substituiertes Alkyl steht, wobei als Substituenten genannt seien: Halogen, Cyano, $COOR^I$, $-CONR^{II}R^{III}$, $-OR^{IV}$, die Oxo-oder Thioxogruppe sowie jeweils gegebenenfalls substituiertes Aryl, Cycloalkyl oder Heterocyclyl; ferner für jeweils gegebenenfalls substituiertes Alkenyl, Alkinyl, Cycloalkyl oder Aryl oder für gegebenenfalls substituiertes und gegebenenfalls anelliertes Heterocyclyl sowie für $-OR^V$ steht; weiterhin
$R^3$ für Wasserstoff steht,
für den Fall das entweder n = 1 ist und A für $-SO_2$-steht oder n = 1 ist und $R^4$ für Cyanalkoximino oder

Arylthio steht,

$R^4$ für Wasserstoff oder für gegebenenfalls substituiertes Alkyl steht, wobei als Substituenten genannt seien: Halogen, Alkoxy, Alkylthio, Alkylsulfinyl, Alkylsulfonyl, Acyloxy, Acylamino, Cyano, $-COOR^I$, jeweils gegebenenfalls substituiertes Aryl, Cycloalkyl oder Heterocyclyl; ferner für jeweils gegebenenfalls substituiertes Alkenyl oder Alkinyl steht; $R^4$ weiterhin für jeweils gegebenenfalls über brücktes und/oder anelliertes, jeweils gegebenenfalls substituiertes Cycloalkyl oder Cycloalkenyl steht sowie für jeweils gegebenenfalls anelliertes, jeweils gegebenenfalls substiuiertes Aryl oder Heterocyclyl, für jeweils gegebenenfalls substituiertes Aryloxy oder Arylthio sowie für Alkoxycarbonyl, für 1-Cyanoalkoximino, $-OR^5$, $-SR^5$ und $NR^6R^7$ steht,

oder

$R^3$ und $R^4$ gemeinsam mit dem Stickstoffatom und gegebenenfalls dem Rest A einen gegebenenfalls substituierten 4- bis 9-gliedrigen, gesättigten oder ungesättigten heterocyclischen Ring bilden, welcher gegebenenfalls weitere Heteroatome aus der Reihe Sauerstoff, Schwefel, Stickstoff enthält und welcher gegebenenfalls durch 1 oder 2 gesättigte oder ungesättigte Carbocyclen anelliert ist,

$R^5$ für Wasserstoff, für gegebenenfalls substituiertes Alkyl steht, wobei als Substituenten genannt seien: Halogen, $-OR^{IV}$, $-SR^{IV}$, $-COOR^I$, $-CONR^{II}R^{III}$, -CN, $-NR^{II}R^{III}$ Acyl, jeweils gegebenenfalls substituiertes Aryl oder Aryloxy, jeweils gegebenenfalls substituiertes Cycloalkyl, Cycloalkenyl oder Heterocyclyl; ferner für jeweils gegebenenfalls substituiertes Alkenyl oder Alkinyl sowie für jeweils gegebenenfalls substituiertes Cycloalkyl oder Cycloalkenyl steht,

$R^6$ für Wasserstoff oder für gegebenenfalls substituiertes Alkyl steht, wobei als Substituenten genannt seien: Halogen, Cyano, $-COOR^I$ $-CONR^{II}R^{III}$, $-NR^{II}R^{III}$, $-OR^{IV}$, $-S(O)_nR^{IV}$, Acyl, jeweils gegebenenfalls substituiertes Aryl, Heterocyclyl, Cycloalkyl oder Cycloalkenyl; ferner für jeweils gegebenenfalls substituiertes Alkenyl, Alkinyl, Cycloalkyl, Cycloalkenyl, Aryl oder Heterocyclyl steht und

$R^7$ für die Bedeutung von $R^6$ oder für $-OR^{IV}$ steht,

oder

$R^6$ und $R^7$ gemeinsam mit dem Stickstoffatom, an das sie gebunden sind, für einen gegebenenfalls substituierten Heterocyclus stehen, der weitere Heteroatome enthalten kann,

$R^I$ für Wasserstoff oder gegebenenfalls substituiertes Alkyl steht mit folgenden Substituenten: Halogen, jeweils gegebenenfalls substituiertes Aryl, Cycloalkyl und Cycloalkenyl,

$R^{II}$ und $R^{III}$ gleich oder verschieden sind und für Wasserstoff oder gegebenenfalls substituiertes Alkyl stehen mit folgenden Substituenten: Halogen, $-COOR^{IV}$, $-CONR^IR^{IV}$, jeweils gegeben falls substituiertes Aryl, Aralkyl, Cycloalkyl und Cycloalkenyl; $R^{II}$ und $R^{III}$ ferner für Alkenyl, Alkinyl oder jeweils gegebenenfalls substituiertes Cycloalkyl, Cycloalkenyl, Aryl oder Heterocyclyl stehen oder gemeinsam mit dem Stickstoffatom, an das sie gebunden sind, für einen gegebenenfalls substituierten Heterocyclus stehen, der weitere Heteroatome enthalten kann,

$R^{IV}$ für Wasserstoff, Alkyl, Aralkyl oder Acyl steht und

$R^V$ für Wasserstoff, Alkyl oder Aralkyl steht, dadurch gekennzeichnet, daß man

a) 2-cyano-2-oximinocarbonyl-Verbindungen der Formel (II)

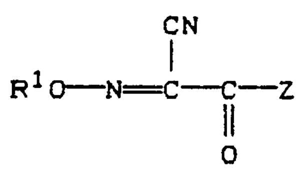

(II)

in welcher

$R^1$ die oben angegebene Bedeutung hat und

Z für Chlor, Methoxy oder Ethoxy steht

mit Amino-Verbindungen der Formel (III)

HY x 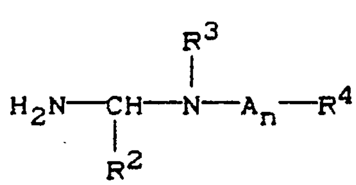 (III)

in welcher

$R^2$, $R^3$, $R^4$, A und n die oben angegebene Bedeutung haben und
HY für das Äquivalent einer anorganischen oder organischen Säure steht,
gegebenenfalls in Gegenwart einer Base und in Gegenwart eines Verdünnungsmittels und gegebenenfalls in Gegenwart eines Katalysators umsetzt;
oder
b) 2-Cyano-2-oximinoacetamide der Formel (IV)

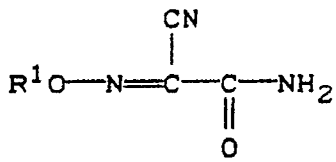

(IV)

in welcher
$R^1$ die oben angegebene Bedeutung hat,
mit Verbindungen der Formel (V),

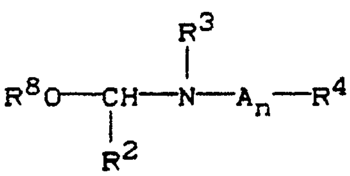

(V)

in welcher
$R^2$, $R^3$, $R^4$, A und n die oben angegebene Bedeutung haben und
$R^8$ für Wasserstoff, Methyl oder Acetyl steht,
gegebenenfalls in Gegenwart einer Säure und gegebenenfalls in Gegenwart eines Verdünnungsmittels umsetzt;
oder
c) 2-Cyano-2-oximinoacetamide der Formel (IV)

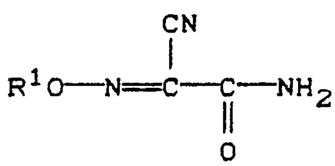

(IV)

in welcher
$R^1$ die oben angegebene Bedeutung hat,
mit Halogen-Verbindungen der Formel (VI)

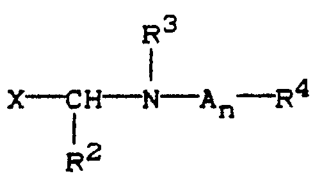

(VI)

in welcher
$R^2$, $R^3$, $R^4$, A und n die oben angegebene Bedeutung haben und
X für Chlor oder Brom steht,
gegebenenfalls in Gegenwart einer Base und gegebenenfalls in Gegenwart eines Verdünnungsmittels umsetzt, oder
d) die erfindungsgemäßen Verbindungen der Formel (Ia)

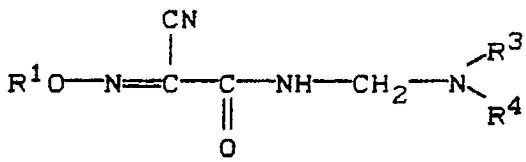

(Ia)

in welcher
$R^1$, $R^3$ und $R^4$ die oben angegebene Bedeutung haben, erhält,
wenn man
2-Cyano-2-oximinoacetamide der Formel (IV)

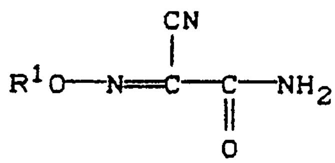

(IV)

in welcher
$R^1$ die oben angegebene Bedeutung hat,
mit Aminen der Formel (VII)

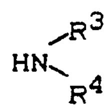

(VII)

in welcher
$R^3$ und $R^4$ die oben angegebene Bedeutung haben und mit Formaldehyd gegebenenfalls in Gegenwart eines Verdünnungsmittels umsetzt, oder

e) die erfindungsgemäßen Verbindungen der Formel (Ib)

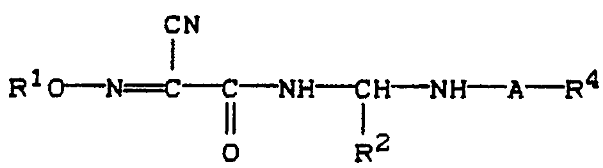

(Ib)

in welcher
$R^1$, $R^2$, $R^4$ und A die oben angegebene Bedeutung haben, erhält, wenn man
N-(2-Cyano-2-oximino-acetyl)-aminale der Formel (VIII)

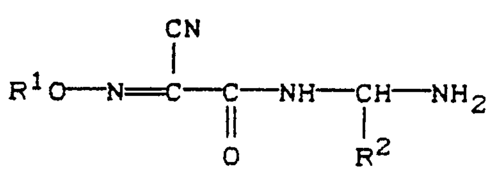

x HY (VIII)

in welcher
$R^1$ und $R^2$ die oben angegebene Bedeutung haben,
und
HY für das Äquivalent einer anorganischen oder organischen Säure steht,
mit einem Acylierungsreagenz der Formel (IX)
$Q\text{-}A\text{-}R^4$ (IX)

in welcher
$R^4$ und A die oben angegebene Bedeutung haben und
Q für eine übliche Abgangsgruppe steht, wie Halogen Alkoxy, Alkylthio, -O-$COR^4$, -O-$COOR^5$, -$OR^5$, -$SR^5$, Carboxymethoxy oder Carboxymethylthio steht,
gegebenenfalls in Gegenwart einer Base und in Gegenwart eines Verdünnungsmittels und gegebenenfalls in Gegenwart eines Katalysators umsetzt.

5. Schädlingsbekämpungsmittel, gekennzeichnet durch einen Gehalt an mindestens einem Aminal der Formel (I) nach den Ansprüchen 1 bis 4.

6. Verwendung von Aminalen der Formel (I) nach den Ansprüchen 1 bis 4 zur Bekämpfung von Schädlingen.

7. Verfahren zur Bekämpfung von Schädlingen, dadurch gekennzeichnet, daß man Aminale der Formel (I) nach den Ansprüchen 1 bis 4 auf Schädlinge und/oder ihren Lebensraum einwirken läßt.

8. Verfahren zur Herstellung von Schädlingsbekämpfungsmitteln, dadurch gekennzeichnet, daß man Aminale der Formel (I) nach den Ansprüchen 1 bis 4 mit Streckmitteln und/oder oberflächenaktiven Mitteln vermischt.